# EUROPÄISCHE PATENTSCHRIFT

(11) **EP 2 672 806 B1**
(45) Veröffentlichungstag und Bekanntmachung des Hinweises auf die Patenterteilung: **19.04.2017**
(21) Anmeldenummer: 12708785.6
(22) Anmeldetag: 10.02.2012
(51) Int. Cl.: A01G 7/00

(54) **TEMPERATUR-UNABHÄNGIGE TURGORDRUCK-MESSEINRICHTUNG, VERFAHREN ZUR HERSTELLUNG DER MESSEINRICHTUNG**
TEMPERATURE-INDEPENDENT TURGOR PRESSURE MEASUREMENT DEVICE, METHOD FOR PRODUCING SAID MEASUREMENT DEVICE
DISPOSITIF DE MESURE DE PRESSION DE TURGESCENCE INDÉPENDANTE DE LA TEMPÉRATURE, PROCÉDÉ DE PRODUCTION DE CE DISPOSITIF DE MESURE

(30) Priorität: 11.02.2011 DE 102011011020; 27.07.2011 DE 102011079905
(43) Veröffentlichungstag der Anmeldung: 18.12.2013
(73) Patentinhaber: YARA ZIM Plant Technology GmbH, 16761 Hennigsdorf (DE)
(72) Erfinder: ZIMMERMANN, Ulrich, 14612 Falkensee (DE); EHRENBERGER, Wilhelm, 10437 Berlin (DE); RÜGER, Simon, 10437 Berlin (DE); ZIMMERMANN, Gertraud, 97295 Waldbrunn (DE)
(74) Vertreter: Vossius & Partner Patentanwälte Rechtsanwälte mbB
(86) Internationale Anmeldenummer: PCT/EP2012/052295
(87) Internationale Veröffentlichungsnummer: WO 2012/107555

(56) Entgegenhaltungen:
- WO-A1-2009/092389
- DE-A1-102006 043 058
- DE-A1-102009 032 872

## Beschreibung

Die Erfindung betrifft ein Klemmelement für eine Turgordruck-Messeinrichtung, eine Turgordruck-Messeinrichtung, ein Verfahren zur Herstellung eines Klemmelements für eine Turgordruck-Messeinrichtung und ein Verfahren zum Betreiben einer Turgordruck-Messeinrichtung.

Die Erfindung macht sich den Umstand zunutze, dass der hydrostatische Überdruck, d. h. der sogenannte Turgordruck, in pflanzlichen Zellen einen der wichtigsten pflanzlichen Parameter zur Erfassung des Pflanzenzustandes darstellt.

Es ist bekannt, dass der Turgordruck über die Wasserversorgung der Pflanzen Auskunft gibt. Bekannt ist ferner, dass der Turgordruck durch osmotische Prozesse, die ihrerseits vom Stofftransport abhängen, aufgebaut wird. Auf diese Weise spiegelt der Turgordruck auch die Versorgung der Pflanzen mit Nährstoffen wieder. Schließlich ist es bekannt, dass der Turgordruck je nach Art der Pflanze ca. 700 kPa (= 7 bar) betragen kann und dass der Turgordruck auf sehr niedrige Werte von etwa 30 kPa-50 kPa abfällt, wenn große Trockenheit (Wasserknappheit) oder hohe Temperaturen bzw. eine niedrige relative Luftfeuchtigkeit eintritt. Bei einem Turgordruck von Null sterben die pflanzlichen Zellen und somit die Pflanze. Ein ausreichend hoher Turgordruck ist weiterhin für das Pflanzenwachstum wichtig, da das Zellwachstum bzw. viele biophysikalische Prozesse in der Pflanze über den Turgordruck gesteuert werden.

Die im Bereich der Landwirtschaft bekannte praktische Bedeutung des Turgordrucks ergibt sich daraus, dass die Qualität von Früchten oftmals davon abhängt, dass Pflanzen unter einen leichten Wasserstress gesetzt werden, der zu einem suboptimalen Turgordruck führt. Bekannte Beispiele dafür sind Rotweinreben oder Oliven. Die Qualität des Rotweins bzw. die Qualität von Oliven wird erheblich verbessert, wenn die Pflanzen bzw. die Bäume nicht ausreichend bewässert werden. Aufgrund dieser skizzierten Bedeutung des Turgordrucks in Pflanzen als Indikator für die Bewässerung wird bereits seit mehr als 60 Jahren versucht, den Turgordruck messtechnisch zu erfassen, um über eine gezielte Bewässerung das für die jeweilige Pflanze jeweils optimale Ernteergebnis zu erzielen.

In Bezug auf den Stand der Technik ist als Methode zur Messung des Turgordrucks in pflanzenphysiologischen Laboratorien die Plasmolyse bekannt, d. h. die Blätter der Pflanze werden abgeschnitten und in Lösungen mit steigender Osmolarität überführt. Aus dem osmotischen Druck, der bei der Plasmolyse auftritt (d. h. bei der Abtrennung der Zellenmembran von der Zellwand), lässt sich der Turgordruck berechnen. Nachteile dieser Methode in der praktischen Anwendbarkeit bestehen jedoch darin, dass sie destruktiv, arbeitsintensiv, sehr ungenau, nicht automatisierbar und für den Einsatz in der Landwirtschaft nicht feldtauglich ist.

Fortschritte in der Bestimmung des Turgordruckes wurden durch die Entwicklung der Zellturgordruck-Messsonde erzielt. Bei dieser Methode wird eine mit inkompressiblem Öl gefüllte Mikrokapillare unter dem Mikroskop in eine Zelle durch das umliegende Gewebe eingeführt und der Druck über die Ölphase auf einen Drucksensor geleitet (vgl. Zimmermann et al., 1969, Kontinuierliche Druckmessung in Pflanzenzellen, Naturwissenschaften 56, 634). Diese minimal-invasive Methode ist hochpräzise.

Bereits bekannt sind unterschiedliche Modifikationen dieses Sondentypes, die in den letzten 40 Jahren entwickelt wurden, aber sich alle auf die Entwicklung der Zellturgordruck-Messsonde zurückführen lassen (Zimmermann et al., 2004, Water ascent in tall trees: does evolution of land plants rely on a highly metastable state? New Phytologist, 575-615).

Ein Nachteil der Zellturgordruck-Methode besteht darin, dass sie sehr diffizil und komplex ist. Außerdem ist sie nicht feldtauglich, da sie insbesondere windempfindlich ist. Langzeitmessungen im Feld sind deshalb nicht möglich.

Eine weitere, auch heute sowohl unter Labor-, wie auch unter Feldbedingungen angewandte Methode zur indirekten Bestimmung von relativen Änderungen des Turgordrucks (bzw. des Wasserpotentials) ist die Druckbombenmethode nach Scholander (Scholander et al., 1965, Sap pressure in vascular plants. Science 148, 339-346; Tyree und Hammel, 1972, The measurement of the Turgor pressure and the water relations of plants by the pressure-bomb technique. Journal of experimental Botany 23, 267-282). Bei dieser Methode wird ein Blatt/Pflanzenorgan abgeschnitten und in einer Druckbombe unter Gasdruck gesetzt. Der Druck, bei dem Wasser an der Schnittstelle, die mit der Atmosphäre in Verbindung steht, austritt, wird numerisch dem Wasserpotential gleichgesetzt. Die Methode ist allerdings arbeitskräfteintensiv, destruktiv, sehr ungenau und lässt sich wie die Methode der Plasmolyse nicht automatisieren.

Eine nicht-invasive Messung zur Erfassung von relativen Änderungen im Turgordruck wurde 1979 von Heathcote et al. beschrieben und 1983 von Turner und Sobrado angewendet (siehe auch in diesem Zusammenhang den Übersichtsartikel von Geitmann, 2006, Experimental approaches used to quantify physical parameters at cellular and subcellular levels. American Journal of Botany 93, 1380-1390). Bei dieser Methode wird über eine Feder ein Druck auf die Zelle ausgeübt und die Deformation bzw. die Elastizität der Zelle, die vom Turgordruck abhängt, gemessen. Nachteile sind die Ungenauigkeit der Methode und ihre Nichtanwendbarkeit für eine ganze Reihe von Pflanzen, sofern die Blätter einer Pflanze sehr starr sind. Eine Automatisierung ist ebenfalls nicht oder nur über einen sehr hohen Aufwand möglich.

Die Methode von Heathcote et al. (1979) ist aus der Patentanmeldung DE 10 2006 043 058 A1 bekannt ("Zustandssensor für Pflanzen sowie Bewässerungsanlage mit einem derartigen Zustandssensor"). Anwendungen dieser Methode in der Praxis haben jedoch gezeigt, dass der Turgordruck nur in wenigen Fällen mit den Werten übereinstimmt, die direkt mit der Zellturgordruck-Messsonde gemessen werden. Die in der Anmeldung angegebenen Messkurven stimmen teilweise nicht mit den realen Bedingungen überein, was erklärt, warum sich die Heathcote-Methode nicht durchgesetzt hat.

Eine weitere Methode zur Turgordruck-Messung wurde von Lintilhac und Outwater am 23. März 1999 in den Vereinigten Staaten von Amerika zum Patent angemeldet (Method and Apparatus for Determining a Contact Area between a probe and a specimen, US 6,277,637 B1). Dieser Patenschrift liegt die Idee zugrunde, dass die Form einer Zelle bzw. der Kontakt zwischen der Zelle und einem Instrument, das die Formveränderung misst, sich mit dem Turgordruck ändert. Dieses Verfahren lässt sich daher nur auf vereinzelte Zellen aber nicht auf Zellen in einem pflanzlichen Gewebe anwenden.

Ferner ist die Patentanmeldung WO 2009/092389 A1 bekannt. Allerdings müssen bei dieser vorgeschlagenen Lösung mehrere Druckpulse nach Anbringen der Sonde ausgeführt werden, um einen homogenen planaren Kontakt zwischen Pflanzenprobe und der Vergussmasse zu erreichen. Dabei kann es grundsätzlich zu Schädigungen am Blatt kommen und die Messung nach wenigen Stunden bzw. Tagen unbrauchbar werden.

Bekannt ist ferner die Patentanmeldung DE 10 2009 032 872 A1, die sich über ein Verfahren zur Herstellung einer Messeinrichtung verhält, die zur Erfassung des Zustandes einer Pflanzenprobe eingerichtet ist und eine Klemmeinrichtung mit einem Sensor-Klemmblock und einem Gegen-Klemmblock zur Ausübung eines Klemmdruckes auf die Pflanzenprobe und eine Sensoreinrichtung mit einem Sensor zur Messung eines Druckresponsesignals der Pflanzenprobe umfasst. Bei diesem Lösungsvorschlag tritt in der Praxis das Problem auf, dass die vorgeschlagene Herstellung nicht für eine Massenproduktion von Sonden verwendet werden kann. Die erforderlichen Arbeitsschritte sind zeitaufwendig und können zum überwiegenden Teil nicht automatisiert werden. Insbesondere der Nullabgleich der Sonde variiert erheblich nach der Einbettung des Sensors in die polymere und vernetzte Matrix. Es treten Spannungswerte zwischen -15 mV und + 15 mV auf, d. h. der Sensor in der polymeren Matrix steht entweder unter einer Zugspannung (bei negativen Spannungswerten) oder unter einem Überdruck (bei positiven Spannungswerten). Der Nullwert ist zudem im geschlossenen Zustand signifikant unterschiedlich zum Nullwert im offenen Zustand (wenn beide Magneten auseinander geklappt sind), da bei diesem Vorschlag die polymere und vernetzte Matrix nicht reproduzierbar planar eingebracht wird. Da die Zellen in einem pflanzlichen Gewebe (Blatt, Stängel etc.) unter einem Überdruck (dem Turgordruck, 30 kPa bis 700 kPa) stehen, kann es beim Einbringen des Blattes oder eines anderen pflanzlichen Gewebes zwischen dem Sensor-Klemmblock und dem Gegen-Klemmblock der Sonde zu einer weiteren, unbekannten Nullpunktverschiebung kommen. Beim vorgenannten Beispiel kommt es beim Zurückgehen auf den Nullwert des Sensors nach Applikation von höheren Druckwerten (> 50 kPa) zu einer Hysterese, da Silikon und Kleber unterschiedliche Rückstelleigenschaften besitzen, die sich verzögert auf die Sensor-Membran auswirken. Das größte Problem der Messeinrichtungen, die nach diesem Verfahren hergestellt sind, ist vor allem die Temperaturabhängigkeit der Sonden nach Montage und Verguss mit dem Polymer, von über 6 kPa pro 10 Grad Celsius Temperaturänderung (Änderungen an der Pflanze von ca. 5-100 kPa). Ein weiterer Nachteil der sich daraus ergibt ist, dass sich der Nullwert mit der Temperatur ändert. Unter Feldbedingungen, besonders in sehr trockenen und warmen Regionen, kann die Temperatur erheblich schwanken (zwischen 15 Grad Celsius in der Nacht und 45 Grad Celsius tagsüber). Blätter im Schatten sind einer anderen Temperatur ausgesetzt als Blätter, die direkt der Sonneneinstrahlung ausgesetzt sind. Dadurch überlagert sich die Verschiebung des Nullpunktes durch Temperatur-Schwankungen mit denen durch Änderungen im Turgordruck und lässt somit eine genaue Aussage über den Wassergehalt der Pflanze nicht zu. Die Temperaturabhängigkeit des Nullwertes wird zum großen Teil durch eine nicht vollständige Polymerisation in der Nähe des Sensors, eine limitierte Adhäsion des Polymers an der Wand des Sondengehäuses, durch nicht vollständig polymerisierte Mikrokavitäten und durch Einschluss von kleinsten Luftblasen hervorgerufen. Die Temperaturabhängigkeit von Sensoren kann zwar über die Span- und Offsetmethode kompensiert werden, Experimente haben aber gezeigt, dass diese Methoden bei den magnetischen Sonden nicht zum Erfolg führen. Ein Nachteil der oben beschriebenen Methode besteht ferner darin, dass die Bonddrähte am Sensor durch unterschiedliche Temperaturausdehnung reißen können, da für das Befestigen der Bonddrähte ein anderes Material als für die Einbettung des Sensors in die Polymermasse verwendet wird. Diese Materialien haben unterschiedliche Ausdehnungskoeffizienten. Nachteil dieser Sonden ist auch, dass ein homogener Kontakt zwischen Sonde und Blatt - wenn überhaupt - nur durch mehrere Druckpulse, wie in WO 2009/092389 A1 beschrieben ist, erreicht werden kann. Für den massenhaften Feldeinsatz ist es erforderlich, dass die Messeinrichtung von einem nicht vorgebildeten Landwirt ohne Spezialkenntnisse angesetzt und benutzt werden kann. Im Ergebnis erfüllen nur wenige Sonden (etwa 1% der hergestellten Sonden), die nach dieser Patentanmeldung hergestellt werden, die Voraussetzung, die an eine exakte Messung des Turgordruckes gestellt werden.

Die bekannten Verfahren sind ferner nachteilig, da dort entweder Druckpulse gesetzt werden müssen (vgl. WO 2009/092389 A1) oder die Sonden eine zu starke Temperaturabhängigkeit haben (vgl. WO 2009/092389 A1 und DE 10 2009 032 872 A1).

Die Aufgabe der vorliegenden Erfindung ist es, ein Klemmelement für eine Turgordruck-Messeinrichtung, eine Turgordruck-Messeinrichtung, ein Verfahren zur Herstellung eines Klemmelements für eine Turgordruck-Messeinrichtung, ein Verfahren zur Herstellung einer Turgordruck-Messeinrichtung und ein Verfahren zum Betreiben einer Turgordruck-Messeinrichtung bereitzustellen, die zum ersten Mal erlauben, Sonden in großer Stückzahl herzustellen, die den Turgordruck ohne Verfälschung durch Temperatureinflüsse messen und von einer ungeübten Person (z.B. Landwirt) leicht, ohne Anwendung von Druckpulsen, auf ein Blatt gesetzt werden können.

Diese Aufgabe wird in den Gegenständen und Verfahren der unabhängigen Ansprüche gelöst. Vorteilhafte Ausführungsformen und Anwendungen der Erfindung ergeben sich aus den abhängigen Ansprüchen.

Unter dem Begriff "Pflanzenprobe" wird erfindungsgemäß eine intakte Pflanze, ein Pflanzenorgan bzw. Pflanzengewebe oder ein abgetrennter Teil einer Pflanze, eines Pflanzenorgans bzw. Pflanzengewebes verstanden.

Hinsichtlich des Sondenaufbaus geht ein Aspekt der Erfindung von dem Grundgedanken aus, an der Oberfläche der Sonde, die mit der Oberfläche der Pflanze in Kontakt kommt, einen homogenen Abschluss in einem Schritt zu erreichen (ohne das der Kontakt über die Applikation von mehreren Druckpulsen erreicht werden muss). Dies soll insbesondere im Wesentlichen temperaturunabhängig sein, das heißt, die Homogenität des Abschlusses ist vorzugsweise auch bei Temperaturschwankungen gewährleistet. Unter "homogener Abschluss" wird erfindungsgemäß beispielsweise eine Oberfläche verstanden, die geeignet oder derart ausgestaltet ist, mit der Oberfläche der Pflanze einen gleichmäßigen Kontakt und/oder eine größtmögliche Kontaktfläche zu erzielen. Beispielsweise passt die Oberfläche der Sonde im Sinne des "Schlüssel-Schloss" Prinzips mit der Oberfläche der Pflanze zusammen, zumindest im Wesentlichen.

Weiter geht die Erfindung von dem Grundgedanken aus, den Sensor der Sonde in einer Polymermasse einzubetten. Dabei wird erfindungsgemäß eine homogene Einbettung erzielt derart, dass einerseits zwischen der Polymermasse und dem umgebenden Metallgehäuse eine im Wesentlichen vollständige, vorzugsweise vollständige, Adhäsion vorliegt, andererseits in der Polymermasse im wesentlichen keine Gasblasen (beispielsweise Luftblasen) vorhanden sind, die Polymermasse also im Wesentlichen gasblasenfrei ist. Um die oben aufgeführten Eigenschaften zu erzielen, muss diese Einbettung erfindungsgemäß in mehreren Schritten erfolgen, d.h. die Polymermasse muss erfindungsgemäß in mehreren Schichten eingebracht und über dem Sensor schichtweise aufgebaut werden. Dadurch wird der Einfluss von Temperaturschwankungen erheblich reduziert bzw. eliminiert.

Gemäß einem ersten Aspekt stellt die Erfindung ein Verfahren zur Herstellung eines Klemmelements für eine Turgordruck-Messeinrichtung zum Messen des Turgordrucks in einer Pflanzenprobe bereit. Das erfindungsgemäß hergestellte Klemmelement weist ein Sensoraufnahmeteil auf, mit einer ersten Kontaktfläche, einer der Kontaktfläche gegenüberliegenden Fläche und einer diese verbindenden Umfangsfläche, wobei das Klemmelement eine Längsachse aufweist, die senkrecht zur ersten Kontaktfläche verläuft, und mit einer Ausnehmung auf der Seite der ersten Kontaktfläche, die zur ersten Kontaktfläche hin offen ist. Ferner weist das Klemmelement einen auf einer Leiterplatte/Stanzgitter angeordneten Drucksensor zur Messung eines Druckantwortsignals der Pflanzenprobe auf, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt. Das erfindungsgemäße Klemmelement wird vorzugsweise mit folgenden Schritten hergestellt:
(a) Befestigen der Leiterplatte/Stanzgitter mit dem darauf angeordneten Drucksensor am Boden der Ausnehmung oder an der Wandung der Ausnehmung;
(b) Einfüllen einer ersten Menge an Vergussmaterial in die Ausnehmung, wobei die erste Menge so bemessen ist, dass lediglich die Leiterplatte/Stanzgitter und der Drucksensor bedeckt sind, sowie ein Hochziehen der Vergussmasse an der unteren Hälfte der Innenwand des Sensorgehäuses stattfinden kann;
(c) nach Vernetzung der ersten Vergussmaterialmenge mit der Leiterplatte/Stanzgitter, dem Drucksensor und der Innenwand, Einfüllen einer zweiten Menge an Vergussmaterial derart, dass unter Berücksichtigung der Ausdehnung bei der Vernetzung der zweiten Vergussmaterialmenge mit der Wand der Ausnehmung die zweite Vergussmaterialmenge unterhalb des oberen Randes der Ausnehmung bleibt; und
(d) nach Vernetzung der zweiten Vergussmaterialmenge mit der Ausnehmungswand, Einfüllen einer dritten Menge an Vergussmaterial, derart dass ein passgenauer Abschluss des Vergussmaterials mit der ersten Kontaktfläche erreicht wird.

Vorzugsweise wird vor dem vollständigen Aushärten der dritten Vergussmaterialmenge eine negative Abschlussstruktur auf der Oberfläche des Vergussmaterials entsprechend einer pflanzlichen Oberflächenstruktur ausgebildet.

Vorzugsweise wird die negative Abschlussstruktur auf der Oberfläche des Vergussmaterials eingeprägt. Besonders bevorzugt wird die Abschlussstruktur mittels eines Gegenklemmelements der Turgordruck-Messeinrichtung, das eine entsprechende Gegenstruktur aufweist, erzeugt.

Gemäß einer alternativen Ausführungsform wird nach dem vollständigen Aushärten der dritten Vergussmaterialmenge eine negative Abschlussstruktur auf der Oberfläche des Vergussmaterials entsprechend einer pflanzlichen Oberflächenstruktur ausgebildet. Diese kann mit dem Gegenklemmelement der Turgordruck-Messeinrichtung, die eine entsprechende Gegenstruktur aufweist, erzeugt werden. Beispielsweise geschieht dies durch Einprägen mit dem Gegenklemmelement der Turgordruck-Messeinrichtung bei einer Temperatur von etwa 30°C für einen Zeitraum von 24 bis 120 Stunden.

Zwischen der dritten Vergussmaterialmenge des Klemmelements und dem Gegenklemmelement kann dabei ein Übertragungsmaterial geklemmt sein.

Die negative Abschlussstruktur ist vorzugsweise im Wesentlichen konkav.

Das Polymer wird somit erfindungsgemäß in drei Schritten in das Gehäuse eingebracht: beim ersten Schritt wird so viel Polymer eingebracht, dass die Leiterplatte/Stanzgitter mit dem Drucksensor ohne Kavitäten eingebettet werden kann und ein homogener Kontakt mit der Innenwand des Sensoraufnahmeteils erhalten wird. Nach der Vernetzung mit der Leiterplatte/Stanzgitter, dem Drucksensor und der Gehäusewand und vollständigem Aushärten des Polymers wird so viel Polymer eingebracht, dass unter Berücksichtigung der Ausdehnung bei der Vernetzung der obere Rand des Gehäuses nicht erreicht wird. Im dritten Schritt wird nach der Vernetzung noch so viel Menge (üblicherweise im Nanoliter-Bereich) an Polymer in das Gehäuse injiziert, das vorzugsweise ein planarer, passgenauer Abschluss des Gehäuses erreicht wird.

Anstelle von Silikon oder Polymeren können für die Verfüllung Silikone bzw. Polymere verwendet werden, die Farbstoffe zur Detektierung von Sauerstoff enthalten, der von dem Blatt der Pflanze abgegeben wird, an der gemessen wird.

Vorzugsweise erfolgt in einem weiteren Schritt der homogene Abschluss der Polymermasse, der nicht planar erfolgt, sondern sich an Formen anlehnt, die pflanzlichen Oberflächen entsprechen (wie zum Beispiel gekrümmte Oberflächen, wie sie bei einem Stängel vorliegen oder bei venenreichen Blättern).

In die Oberfläche des magnetischen Gegenstempels werden erfindungsgemäß Strukturen eingebracht, z.B. eingefräst (z.B. konzentrische Ringe). Bei einem eventuellen Überdruck im vernetzten Polymer kann sich dann die relativ elastische Polymermasse über die strukturierten Vertiefungen im Gegenstempel ausdehnen, mit der Folge, dass der Überdruck abgebaut wird und sich ein stabiler Nullpunkt einreguliert, der sich aufgrund der strukturierten Ausdehnungsflächen im Gegenstempel nicht bei Temperaturänderung verschiebt und mit dem gleichzeitig ein homogener Kontakt zwischen den beiden Magneten und einem dazwischen geklemmten Blatt erreicht wird.

Gemäß einer bevorzugten Ausführungsform wird die erfindungsgemäße Sonde während und nach Auspolymerisation schnellen Temperaturwechseln von maximal 110°C und minimal - 10°C ausgesetzt, um eventuelle Spannungen im Material und strukturelle Inhomogenitäten im Polymer zu lösen. Die Periode dieser Temperaturwechsel beträgt beispielsweise 10 min. Während dieses Vorganges ist die Sonde an ein Messgerät, etwa ein Voltmeter angeschlossen, und der Vorgang wird solange durchgeführt, bis sich ein stabiler Wert in Form einer konstanten Spannung einstellt.

Gemäß einem alternativen Aspekt stellt die Erfindung ein Klemmelement für eine Turgordruck-Messeinrichtung zum Messen des Turgordrucks in einer Pflanzenprobe bereit, wobei das Klemmelement ein Sensoraufnahmeteil, ein erstes Kraftelement und einen auf einer Leiterplatte/Stanzgitter angeordneten Drucksensor aufweist. Das Sensoraufnahmeteil hat eine erste Kontaktfläche, eine der Kontaktfläche gegenüber liegende Fläche und eine diese verbindende Umfangsfläche. Das Klemmelement weist eine Längsachse auf, die senkrecht zur ersten Kontaktfläche verläuft. Außerdem hat das Klemmelement eine Ausnehmung auf der Seite der ersten Kontaktfläche, wobei die Ausnehmung zur ersten Kontaktfläche hin offen ist, und wobei das Sensoraufnahmeteil einen Durchgangskanal aufweist, der sich vom Inneren der Ausnehmung zu einer Außenfläche erstreckt. Das erste Kraftelement ist auf der der ersten Kontaktfläche gegenüber liegenden Fläche des Sensoraufnahmeteils angeordnet. Der auf einer Leiterplatte/Stanzgitter angeordnete Drucksensor ist eingerichtet zur Messung eines Druckantwortsignals der Pflanzenprobe. Die Leiterplatte/Stanzgitter ist mit dem Drucksensor derart in der Ausnehmung des Sensoraufnahmeteils angeordnet, dass der Drucksensor der ersten Kontaktfläche zugewandt ist und die Leiterplatte/Stanzgitter auf dem Boden der Ausnehmung liegt. Ferner weist die Leiterplatte/Stanzgitter einen Durchgangskanal auf, der von der der Ausnehmungsöffnung zugewandten Seite der Leiterplatte/Stanzgitter zu der dem Boden der Ausnehmung zugewandten Seite verläuft, wobei die Öffnung des Durchgangskanals auf der dem Boden zugewandten Seite mit der inneren Öffnung des Durchgangskanals des Sensoraufnahmeteils fluchtet. Die Ausnehmung ist mit einem ausgehärteten Vergussmaterial mit homogenem Abschluss an der ersten Kontaktfläche erfüllt.

Gemäß einer bevorzugten Ausführungsform weist die Leiterplatte/Stanzgitter mindestens zwei Durchgangskanäle auf, die von der der Ausnehmungsöffnung zugewandten Seite der Leiterplatte/Stanzgitter zu der dem Boden der Ausnehmung zugewandten Seite der Leiterplatte/Stanzgitter verlaufen.

Es ist ferner bevorzugt, dass das Sensoraufnahmeteil mindestens zwei Durchgangskanäle aufweist, die sich vom Inneren der Ausnehmung zu einer Außenfläche erstrecken, wobei die Durchgangskanal-Öffnungen auf der dem Ausnehmungsboden zugewandten Seite der Leiterplatte/Stanzgitter mit inneren Öffnungen der Durchgangskanäle des Sensoraufnahmeteils fluchten.

Die Löcher dienen zum Einbringen beispielsweise eines Vergusspolymers und Austreten des Polymers, um einen kompletten planaren Verguss mit Polymer und Matrix vernetzenden Substanzen erlauben, wie bereits oben erläutert.

Vorzugsweise weist die den Drucksensor tragende Leiterplatte/Stanzgitter einen thermischen Ausdehnungskoeffizienten in x- und y-Richtung von weniger als 12 x 10⁻⁶ K⁻¹, insbesondere weniger als 16 x 10⁻⁶ K⁻¹ auf. In z-Richtung weist die Leiterplatte/Stanzgitter vorzugsweise einen thermischen Ausdehnungskoeffizienten von weniger als 40 x 10⁻⁶ K⁻¹, insbesondere weniger als 60 x 10⁻⁶ K⁻¹, auf. Die Leiterplatte/Stanzgitter weist in z-Richtung einen geringen Ausdehnungskoeffizienten auf, da die Ausdehnung in z-Richtung die Oberfläche des Sensors und somit den Kontakt zur Pflanze temperaturabhängig beeinflussen würde.

Der Drucksensor ist vorzugsweise auf die Leiterplatte/Stanzgitter aufgeklebt. Erfindungsgemäß ist für die Befestigung des Sensors auf der Leiterplatte/Stanzgitter maximal 10 nl an Klebstoff (z.B. ACC Silicone AS5720) zu verwenden, der einen vergleichbaren oder kleineren Ausdehnungskoeffizient wie das Einbettungspolymer aufweist.

Der Querschnitt der Leiterplatte/Stanzgitter entspricht vorzugsweise dem Querschnitt der Ausnehmung. Dies ermöglicht, dass sie in das umgebende Gehäuse passgenau eingebracht werden kann. Eine Zentrierung des Sensors unter dem Mikroskop ist dann nicht mehr erforderlich.

Vorzugsweise ist in dem Sensoraufnahmeteil zwischen dem Boden oder der Wandung der Ausnehmung und der dem Boden / Wandung zugewandten Seite der Leiterplatte/Stanzgitter eine Klebstoffschicht. Das Material des Klebstoffes weist dabei einen ähnlichen Ausdehnungskoeffizienten auf, wie das Vergussmaterial.

Vorzugsweise ist das Vergussmaterial undurchlässig ist für ultraviolette Strahlung. Gemäß einer weiter bevorzugten Ausführungsform enthält das Vergussmaterial einen Farbstoff, der undurchlässig ist für ultraviolette Strahlung. Dies hat den Zweck, die Lichtabhängigkeit des Siliziumchips des Sensors zu eliminieren oder zumindest zu reduzieren, so dass das gemessene Drucksignal nicht durch einfallendes Licht verfälscht wird. Der Farbstoff kann vor Vermischung mit dem Vergussmaterial für einen Tag bei einem Unterdruck von mindestens 700 kPa entgast werden. Das auf diese Weise entgaste Polymer wird sodann unter Vakuum bzw. bei einem Unterdruck von mindestens 700 kPa in das Gehäuse des Sensor-Klemmelements gefüllt, welches die Leiterplatte/Stanzgitter enthält.

Die Wandungen der Ausnehmung des Sensoraufnahmeteils weisen vorzugsweise keinen Haftvermittler auf, sind also haftvermittlerfrei.

Gemäß einem weiteren Aspekt stellt die Erfindung eine Turgordruck-Messeinrichtung zum Messen des Turgordrucks in einer Pflanzenprobe bereit. Diese weist ein erstes Klemmelement und ein zweites Klemmelement auf. Das erste Klemmelement ist ein Klemmelement gemäß dem ersten Aspekt der Erfindung. Das erste Klemmelement weist ein Sensoraufnahmeteil auf, mit einer ersten Kontaktfläche, einer der Kontaktfläche gegenüberliegenden Fläche und einer diese verbindenden Umfangsfläche. Das Klemmelement weist eine Längsachse auf, die senkrecht zur ersten Kontaktfläche verläuft, sowie eine Ausnehmung auf der Seite der ersten Kontaktfläche, wobei die Ausnehmung zur ersten Kontaktfläche hin offen ist. Ferner weist das Klemmelement ein erstes Kraftelement auf, das auf der der ersten Kontaktfläche gegenüber liegenden Fläche des Sensoraufnahmeteils angeordnet ist. Außerdem ist ein auf einer Leiterplatte/Stanzgitter angeordneter Drucksensor zur Messung eines Druckantwortsignals der Pflanzenprobe vorgesehen, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt. Die Ausnehmung ist mit einem ausgehärteten Vergussmaterial mit homogenem Abschluss an der ersten Kontaktfläche verfüllt. Das zweite Klemmelement weist ein Gegenklemmteil mit einer zweiten Kontaktfläche, einer der zweiten Kontaktfläche gegenüberliegenden Fläche und einer diese miteinander verbindenden Umfangsfläche, sowie ein zweites Kraftelement auf, das auf der der zweiten Kontaktfläche gegenüberliegenden Seite angeordnet ist. Eines der beiden Kraftelemente weist einen Magneten auf. Außerdem ist die Turgordruck-Messeinrichtung bzw. deren Messsignal als Ganzes temperaturkompensiert.

Die erfindungsgemäße Lösung der Temperaturkompensation ist vorteilhaft, da sie auf die Sonde bzw. die Messeinrichtung als Ganzes angewendet wird. Im Stand der Technik wird lediglich der Sensorchip kompensiert.

Auch die Temperaturkompensation der Turgordruck-Messeinrichtung bzw. deren Messsignal als Ganzes erfolgt vorzugsweise mittels linearer Regression. Dazu kann beispielsweise in einem ersten Schritt eine Regressionsgerade für die Temperaturabhängigkeit des Ausgangssignals der Messeinrichtung ermittelt werden, und anschließend ein kompensiertes Drucksignal berechnet werden. Beispielsweise erfolgt dies gemäß der Gleichung V[p] = V[p, T] - ((T · m) + t), wobei V[p, T] das von Druck und Temperatur abhängige Ausgangssignal, T die Temperatur, m die Steigung der Regressionsgeraden und t der y-Achsenabschnitt der Regressionsgeraden ist.

Alternativ zu dieser softwarebasierten Temperaturkompensation kann die Kompensation hardwarebasiert erfolgen. Dazu kann zunächst wiederum die Regressionsgerade für die Temperaturabhängigkeit des Ausgangssignals der Messeinrichtung ermittelt werden, um dann eine elektrisches Bauteil, wie einen Widerstand oder eine Diode, zum Ausgangssignal des Drucksensorchips der Messeinrichtung in Serie zu schalten, wobei das elektrische Bauteil eine der Regressionsgeraden entgegen gesetzte Temperaturabhängigkeit hat.

Die erfindungsgemäße Turgordruck-Messeinrichtung bzw. Sonde besteht somit aus einem Sensor-Klemmblock und einem Gegen-Klemmblock. In den Sensor-Klemmblock ist der Sensor, vorzugsweise ein Drucksensor, eingebettet. Die beiden Klemmblöcke bestehen vorzugsweise aus einem metallischen Material.

Sowohl bei dem Klemmelement als auch bei der Turgordruck-Messeinrichtung kann das Sensoraufnahmeteil einen Durchgangskanal aufweisen, der sich vom Inneren der Ausnehmung zu einer Außenfläche erstreckt, und die Leiterplatte/Stanzgitter einen Durchgangskanal aufweisen, der von der der Ausnehmungsöffnung zugewandten Seite der Leiterplatte/Stanzgitter zu der dem Boden der Ausnehmung zugewandten Seite verläuft. Die Öffnung des Durchgangskanals auf der dem Boden zugewandten Seite fluchtet dabei mit der inneren Öffnung des Durchgangskanals des Sensoraufnahmeteils. Dadurch ist es möglich, beim Verfüllen bzw. Vergießen der Polymermasse die enthaltene Luft entweichen zu lassen, so dass es nicht zu Lufteinschlüssen kommt.

Gemäß einer bevorzugten Ausführungsform weist die Leiterplatte/Stanzgitter mindestens zwei Durchgangskanäle auf, die von der der Ausnehmungsöffnung zugewandten Seite der Leiterplatte/Stanzgitter zu der dem Boden der Ausnehmung zugewandten Seite der Leiterplatte/Stanzgitter verlaufen.

Es ist ferner bevorzugt, dass das Sensoraufnahmeteil mindestens zwei Durchgangskanäle aufweist, die sich vom Inneren der Ausnehmung zu einer Außenfläche erstrecken, wobei die Durchgangskanal-Öffnungen auf der dem Ausnehmungsboden zugewandten Seite der Leiterplatte/Stanzgitter mit inneren Öffnungen der Durchgangskanäle des Sensoraufnahmeteils fluchten.

Die Löcher dienen zum Einbringen beispielsweise des Vergusspolymers (z. B. Silikon; SYLGARD® 186 SILICONE ELASTOMER KIT) und Austreten des Polymers, um einen kompletten, planaren Verguss mit Polymer und Matrix vernetzenden Substanzen erlauben. So weist das Vergussmaterial z.B. ein vernetzbares Polymer auf oder besteht aus vernetzbarem Polymer. Beispielweise ist das Vergussmaterial ausgewählt aus Epoxydharz, Silikon, additiv vernetzendem Silikon, kondensierend vernetzendem Silikon, organischem Hydrogel oder natürlichem Hydrogel mit einer Wärmeleitfähigkeit >0,4 Watt x m⁻¹ x K⁻¹. Es werden erfindungsgemäß vorzugsweise (konkave Menisken bildende) Polymere verwendet, die sich aufgrund ihrer Oberflächenspannungs-Eigenschaften beim Einbringen in das Gehäuse an den Innenwänden des Sensor-Klemmblockes hochziehen und somit einen homogen Kontakt mit diesen herstellen. Es ist ferner die optimale Temperatur in Abhängigkeit von der verwendeten Vergussmasse zu berücksichtigen, bei der die Oberflächenspannungseffekte die oben aufgeführten Eigenschaften aufweisen. Die Polymerisierung muss deshalb in einem engen Temperaturintervall durchgeführt werden (z.B. zwischen etwa 20°C und etwa 23°C).

Vorzugsweise weist die den Drucksensor tragende Leiterplatte/Stanzgitter einen thermischen Ausdehnungskoeffizienten in x- und y-Richtung von weniger als 12 x 10⁻⁶ K⁻¹, insbesondere weniger als 16 x 10⁻⁶ K⁻¹ auf. In z-Richtung weist die Leiterplatte/Stanzgitter vorzugsweise einen thermischen Ausdehnungskoeffizienten von weniger als 40 x 10⁻⁶ K⁻¹, insbesondere weniger als 60 x 10⁻⁶ K⁻¹, auf. Die Leiterplatte/Stanzgitter weist in z-Richtung einen geringen Ausdehnungskoeffizienten auf, da die Ausdehnung in z-Richtung die Oberfläche des Sensors und somit den Kontakt zur Pflanze temperaturabhängig beeinflussen würde.

Der Drucksensor ist vorzugsweise auf die Leiterplatte/Stanzgitter aufgeklebt. Erfindungsgemäß ist für die Befestigung des Sensors auf der Leiterplatte/Stanzgitter maximal 10 nl an Klebstoff (z.B. ACC Silicone AS5720) zu verwenden, der einen vergleichbaren oder kleineren Ausdehnungskoeffizient wie das Einbettungspolymer aufweist.

Der Querschnitt der Leiterplatte/Stanzgitter entspricht vorzugsweise dem Querschnitt der Ausnehmung. Dies ermöglicht, dass sie in das umgebende Gehäuse passgenau eingebracht werden kann. Eine Zentrierung des Sensors unter dem Mikroskop ist dann nicht mehr erforderlich.

Vorzugsweise ist in dem Sensoraufnahmeteil zwischen dem Boden oder der Wandung der Ausnehmung und der dem Boden / Wandung zugewandten Seite der Leiterplatte/Stanzgitter eine Klebstoffschicht. Das Material des Klebstoffes weist dabei einen ähnlichen Ausdehnungskoeffizienten auf, wie das Vergussmaterial.

Vorzugsweise ist das Vergussmaterial undurchlässig für ultraviolette Strahlung. Gemäß einer weiter bevorzugten Ausführungsform enthält das Vergussmaterial einen Farbstoff, der undurchlässig ist für ultraviolette Strahlung. Dies hat den Zweck, die Lichtabhängigkeit des Siliziumchips des Sensors zu eliminieren oder zumindest zu reduzieren, so dass das gemessene Drucksignal nicht durch einfallendes Licht verfälscht wird. Der Farbstoff kann vor Vermischung mit dem Vergussmaterial für einen Tag bei einem Unterdruck von mindestens 700 kPa entgast werden. Das auf diese Weise entgaste Polymer wird sodann unter Vakuum bzw. bei einem Unterdruck von mindestens 700 kPa in das Gehäuse des Sensor-Klemmelements gefüllt, welches die Leiterplatte/Stanzgitter enthält.

Falls die Wände des Gehäuses nicht durch Oberflächenspannungseffekte mit dem Polymer überzogen werden, werden die Wände des Gehäuses gemäß einer bevorzugten Ausführungsform vor dem Einbringen des Polymers mit einem leichten, stabilen Film des Polymers (zum Beispiel durch Zentrifugieren) überzogen, so dass es bei der Vernetzung des Polymers nicht zu Kavitäten zwischen dem Polymer und den Innenwänden des Sensor-Klemmblockes kommt. Vorzugsweise erfolgt das Einfüllen des Einbettungspolymers in mehreren Schritten, um einen homogenen Kontakt zwischen Innenwand und Polymer zu erhalten. Beim ersten Verfüllungsschritt muss darauf geachtet werden, dass sich das noch flüssige Polymer aufgrund von Oberflächenspannungseffekten homogen an der Wand des Gefäßes hochzieht, so dass eine homogene Adhäsion zwischen Polymer und Wand entsteht.

Bei der Turgordruck-Messeinrichtung ist die zweite Kontaktfläche des zweiten Klemmelements vorzugsweise strukturiert. Die Struktur kann dabei in Form eines Reliefs vorliegen. Die Struktur kann etwa in Form von konzentrischen Ringen vorliegen. Dies trägt dazu bei, dass der Kontakt zur Pflanzenprobe erhöht wird, da die Ringe der Oberfläche eine gewisse Elastizität verleihen. Es ist ferner bevorzugt, dass die Struktur in Form von parallelen Nuten vorliegt, wobei die am nächsten an der Längsachse gelegene Nut tiefer ist als die übrigen Nuten. Bei einem eventuellen Überdruck im vernetzten Polymer kann sich dann die relativ elastische Polymermasse über die strukturierten Vertiefungen im Gegenstempel ausdehnen, mit der Folge, dass der Überdruck abgebaut wird und sich ein stabiler Nullpunkt einreguliert, der sich aufgrund der strukturierten Ausdehnungsflächen im Gegenstempel nicht bei Temperaturänderung verschiebt und mit dem gleichzeitig ein homogener Kontakt zwischen den beiden Magneten und einem dazwischen geklemmten Blatt erreicht wird. Parallele Nuten sind für die Messung an Nadeln geeignet oder für Blätter, wobei die mittige tiefere Nut die Mittelrippe des Blattes aufnehmen kann. Allgemein gesagt wird ein gleichmäßiger und größtmöglicher Kontakt bereitgestellt.

Aus der Regressionsgeraden für die Temperaturkompensation wird demnach zunächst die Steigung (m) sowie der y-Achsenabschnitt (t) bestimmt. Um die Temperaturabhängigkeit zu kompensieren, wird V [p] (kompensiertes Ausgangssignal) nach der Gleichung V[p] = V[p, T] - ((T · m) + t) berechnet. Da das Sondensignal gemäß der vorgenannten Gleichung auch vom Druck abhängt, wird anschließend die Druckabhängigkeit der Sonde bestimmt, indem die Sonde gegen den Luftdruck geeicht wird. Das temperaturkompensierte Signal V [p] wird mit einer entsprechenden Software in der Datenbank abgespeichert und vorzugsweise entsprechend visualisiert.

Schritt (a) weist dabei vorzugsweise die Schritte auf:
(a1) Beaufschlagen der Messeinrichtung mit einem stufenweisen Temperaturanstieg in Stufen von 10° C von 0° C bis 50° C im Abstand von jeweils mindestens 30 min.;
(a2) Messen des Ausgangssignals der Messeinrichtung an der jeweiligen Stufe; und
(a3) Ermitteln der Regressionsgeraden aus den Messwerten.

Bei dieser Softwarelösung wird der lineare Zusammenhang zwischen Temperatur und Ausgangssignal genutzt. Dabei wird die Temperatur alle 30 Minuten um jeweils 10 Grad Celsius von 0 bis 50 Grad Celsius erhöht und zugleich werden die Ausgangssignale der Sonden aufgezeichnet.

Ferner erfolgt vorzugsweise eine Eichung der Sonde gegen den Luftdruck und ein Speichern des temperaturkompensierten Signals V[p] für jede Messeinrichtung in einer Datenbank.

Bei dieser Temperaturkompensation erfolgt vorzugsweise ein "preconditioning". Dabei erfolgt zunächst eine Abkühlung auf 0°C bis zum Erreichen stabiler Werte (mindestens 10 Minuten keine Wertschwankungen im Rahmen der Messgenauigkeit und des natürlichen Rauschens), sowohl vom unkompensierten Ausgangssignal, als auch von der Temperaturmessung. In diesem Zusammenhang sind Vergussmassen mit hohen Wärmeleitfähigkeiten zu bevorzugen, da sie die Einstellung des Gleichgewichts zwischen Sensor und Umgebung beschleunigen.

Die Temperaturmessung für die Kompensation kann auf mehrere Arten erfolgen. Zum einen kann die Messung der Temperatur mittels einer Diode im Sensorgehäuse (z.B. auf Leiterplatte/Stanzgitter) oder über den Gesamtbrückenwiderstand der Wheatstoneschen Brücke (Anschlüsse der Versorgungsspannung V+ und GND) erfolgen. Dieser Gesamtbrückenwiderstand ist temperaturabhängig, aber nicht druckabhängig. Alternativ dazu kann dazu ein Drucksensorchip mit integrierter Temperaturdiode verwendet werden.

Beispielsweise werden zunächst unkompensierte Messwerte ermittelt. Anschließend erfolgt ein Umschalten der Messvorrichtung in den Druckmessmodus, um eine Druckmessung durchzuführen. Dann wird wieder in den Temperaturmodus zur Temperaturmessung umgeschaltet. Nun erfolgt die Kompensation des Druckwertes. Zwischen Druck und Temperaturmessung liegen beispielsweise ca. 1-2 ms.

Alternativ dazu erfolgt die Temperaturkompensation über Hardware. Dies kann auf zwei verschiedene Arten erfolgen. Dabei wird berücksichtigt, dass die Leiterplatte/Stanzgitter neben dem Drucksensor eine Messbrücke, beispielsweise eine Wheatstonesche Brücke aufweist.

Gemäß einer Alternative wird bei geschlossenen Gliedern der Wheatstoneschen Brücke (aber auch im offenen Zustand) die Temperaturabhängigkeit durch adäquate Ersatzschaltungen (z.B. durch parallele oder in Serie geschaltete Widerstände, Dioden etc.) kompensiert. Dazu wird zunächst eine Regressionsgerade für die Temperaturabhängigkeit des Ausgangssignals der Messeinrichtung ermittelt. Anschließend erfolgt beispielsweise ein In-Serie-Schalten eines elektrischen Bauteils zum Ausgangssignal des Drucksensorchips der Messeinrichtung, wobei das elektrische Bauteil eine der Regressionsgeraden entgegengesetzte Temperaturabhängigkeit hat.

Alternativ könnten auch die einzelnen Glieder der Messbrücke verändert werden falls diese offen, d.h. die Wheatstonesche Brücke nicht geschlossen und die Einzelwiderstände zugänglich sind. In jedem Fall muss die Kompensationsvorrichtung in enger räumlicher Nähe liegen, um unterschiedliche Temperaturen der Sonde und des Kompensationswiderstandes zu vermeiden. Das Ausgangssignal ist anschließend dauerhaft kompensiert. Eine Temperaturmessung ist überflüssig, da sich der "Korrekturwiderstand" mit Temperaturänderung mit verändert.

Beispielsweise ist mindestens ein Glied oder alle druckabhängigen Glieder der Wheatstoneschen Brücke des Drucksensors offen, so dass sie über Laserabtragung oder andere Methoden so verändert werden können, mit der Folge, dass die Temperaturabhängigkeit in einem großen Bereich kompensiert wird.

Bei Verwendung der erfindungsgemäßen Sonde werden auftretende Temperaturschwankungen vorzugsweise dadurch kompensiert, dass der Drucksensor in einer Doppelfunktion auch zur Temperaturmessung eingesetzt wird. Dazu wird der Sensor kontinuierlich zwischen Druckmessung und Temperaturmessung umgeschaltet, beispielsweise im Rhythmus von 1-2 ms.

So betrifft ein weiterer erfindungsgemäßer Aspekt ein Verfahren zum Betreiben einer Turgordruck-Messeinrichtung zum Messen des Turgordrucks in einer Pflanzenprobe, mit
(a) einem ersten Klemmelement, das aufweist:
   - ein Sensoraufnahmeteil, mit einer ersten Kontaktfläche, einer der Kontaktfläche gegenüberliegenden Fläche und einer diese verbindenden Umfangsfläche, wobei das Klemmelement eine Längsachse aufweist, die senkrecht zur ersten Kontaktfläche verläuft, und mit einer Ausnehmung auf der Seite der ersten Kontaktfläche, wobei die Ausnehmung zur ersten Kontaktfläche hin offen ist;
   - ein erstes Kraftelement, das auf der der ersten Kontaktfläche gegenüberliegenden Fläche des Sensoraufnahmeteils angeordnet ist;
   - einen auf einer Leiterplatte/Stanzgitter angeordneten Drucksensor zur Messung eines Druckantwortsignals der Pflanzenprobe, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt;
   - wobei die Ausnehmung mit einem ausgehärteten Vergussmaterial mit homogenem Abschluss an der ersten Kontaktfläche verfüllt ist;
(b) einem zweiten Klemmelement, das aufweist:
   - ein Gegenklemmteil mit einer zweiten Kontaktfläche, einer der zweiten Kontaktfläche gegenüberliegenden Fläche und einer diese miteinander verbindenden Umfangsfläche;
   - ein zweites Kraftelement, das auf der der zweiten Kontaktfläche gegenüberliegenden Seite angeordnet ist; wobei das Verfahren die Schritte aufweist:
      abwechselndes Messen eines Druckantwortsignals von der Pflanzenprobe in einem Druckmessbetriebsmodus der Messeinrichtung und Messen der Temperatur der Oberfläche der Pflanzenprobe in einem Temperaturbetriebsmodus der Messeinrichtung.

Damit wird die Temperatur direkt an der Oberfläche des Blattes bzw. des pflanzlichen Gewebes mit Hilfe der Sonde erfasst, so dass die Temperaturabhängigkeit der Sonde über eine entsprechende Hard- und/oder Software kompensiert werden kann. Das Drucksignal kann dadurch hinsichtlich der Temperaturabhängigkeit kompensiert werden.

Dabei wird der Druckmesssensor abwechselnd in einem Druckmessbetriebsmodus und einem Temperaturmessbetriebsmodus betrieben. Gemäß einer bevorzugten Ausführungsform wird kontinuierlich zwischen dem Druckmessbetriebsmodus und dem Temperaturmessbetriebsmodus umgeschaltet. Beispielsweise erfolgt dies in Intervallen von 1-2 ms.

Die Erfindung ermöglicht auf diese Weise eine Reihe von neuen Anwendungen.

So ermöglich die Erfindung ein Verfahren zum Betreiben eines Systems bestehend aus mehreren Turgordruck-Messeinrichtungen zum Messen des Turgordrucks an einer Pflanzenprobe. Die mehreren Turgordruck-Messeinrichtungen sind an der gleichen Pflanzenprobe angebracht und weisen Abstandssensoren zum Ermitteln der Abstände der Messeinrichtungen zueinander auf. Das Verfahren weist die Schritte auf: Erfassen der Abstände der Messeinrichtungen paarweise zueinander an zwei aufeinanderfolgenden Zeitpunkten; und Vergleichen der erfassten Abstandswerte jeweils für einzelne Messeinrichtungs-Paare zur Ermittlung des Pflanzenwachstums.

So können mehrere Sonden elektrisch miteinander gekoppelt werden, so dass beim Anbringen der Sonden an verschiedenen Stellen der Pflanze (beispielsweise längs des Stängels) die Druckübertragung bzw. die Flussgeschwindigkeit oder Luftembolien (Kavitationen) zwischen den Sondenstellen gemessen werden können. Zusätzlich wird hierzu ein Abstandssensor in die Sensoren eingebracht, die somit telemetrisch die Entfernungen der Sensoren untereinander bereitstellen. Über die Abstandsmessungen ist des Weiteren eine Quantifizierung des Pflanzenwachstums möglich.

Durch RFID Chips können die Daten der Sonden zudem kabellos übertragen werden.

Ein anderes Verfahren zum Betreiben eines Systems aus mehreren Turgordruck-Messeinrichtungen zum Messen des Turgordrucks in einer Pflanzenprobe, die an der gleichen Pflanzenprobe angebracht sind, und wobei eine Messeinrichtung und/oder eine getrennte Einrichtung einen ansteuerbaren Elektromagneten aufweist, weist die Schritte auf:
- Aufbringen eines Druckimpulses auf die Pflanzenprobe mittels des Elektromagneten;
- Erfassen, an den Messeinrichtungen, des sich in der Pflanzenprobe ausbreitenden Druckimpulses;
- Ermitteln, aus dem zeitlichen Abstand zwischen Druckimpuls-Auslösung und - Erfassung an den Messeinrichtungen für jede Messeinrichtung und dem örtlichen Abstand zwischen der den Druckimpuls auslösenden Messeinrichtung / Einrichtung und der erfassenden Messeinrichtung, der Ausbreitungsgeschwindigkeit des Druckimpulses; und
- Ermitteln, aus der Ausbreitungsgeschwindigkeit des Druckimpulses, des Anteils an wasser- und luftgefüllten Räumen im Gewebe der Pflanzenprobe.

In einer bevorzugten Ausführungsform können durch die Verwendung von Elektromagneten transiente Druckpulse auf das Blatt lokal ausgeübt werden, so dass die Fortpflanzung- und Ausbreitungsgeschwindigkeit des Druckes im Blatt gemessen werden kann. Darüber lassen sich Informationen über den Anteil an wasser- und luftgefüllten Räumen in dem pflanzlichen Gewebe erhalten.

Eine weitere Anwendung der Erfindung betrifft ein System zur Pflanzenbewässerung mit mehreren Turgordruck-Messeinrichtungen zum Messen des Turgordrucks in einer Pflanzenprobe und einer Messeinrichtung zum Erfassen der Aktivität von Staaten bildenden Bestäubern, wobei bei Erfassung einer Aktivität der Staaten bildenden Bestäuber und gleichzeitiger Erfassung von Wassermangel in der Pflanze die Bewässerung aktiviert wird.

Auf diese Weise kann die erfindungsgemäße Sondentechnik mit einer Messvorrichtung zum Bestäuberflug von Staaten bildenden Bestäubern (Bienen, Hummeln etc.) oder anderen bestäubenden Tieren, d.h. einer Messvorrichtung, die die Aktivität der Bestäuber misst, gekoppelt werden.

Die erfindungsgemäßen Messeinrichtungen lassen sich auch in Kombination mit Bodenfeuchtemessgeräten, Dendrometern, Saftfluss-, Photosynthese-, Chlorophyll-, Licht-, Wind-, Temperatur- und Feuchtigkeitssensoren sowie Sonden, die die Wassermenge pro Zeiteinheit in Bewässerungsschläuchen (Durchflusszähler für Wasser und Wasser plus Dünger, Zusatzstoffe etc.) messen, verwenden.

Die Erfindung wird nachfolgend unter Bezug auf die Zeichnungen näher erläutert. Es zeigen:
- Fig. 1: eine schematische Schnittdarstellung der erfindungsgemäßen Messeinrichtung gemäß einer bevorzugten Ausführungsform;
- Fig. 2: eine schematische Schnittdarstellung der erfindungsgemäßen Messeinrichtung gemäß einer anderen bevorzugten Ausführungsform;
- Fig. 3: eine schematische Schnittdarstellung der erfindungsgemäßen Messeinrichtung gemäß einer anderen bevorzugten Ausführungsform;
- Fig. 4: ein Beispiel einer Eichgeraden für die Temperaturkompensation;
- Fig. 5: eine schematische Schnittdarstellung der erfindungsgemäßen Messeinrichtung gemäß einer weiteren bevorzugten Ausführungsform;
- Fig. 6: eine schematische Schnittdarstellung der erfindungsgemäßen Messeinrichtung gemäß einer weiteren bevorzugten Ausführungsform; und
- Fig. 7: eine schematische Schnittdarstellung der erfindungsgemäßen Messeinrichtung gemäß einer weiteren bevorzugten Ausführungsform.

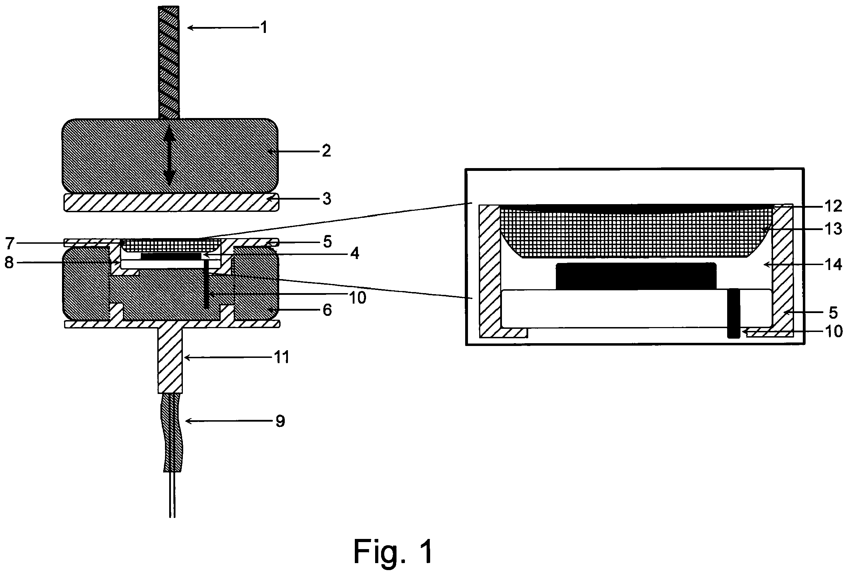

Fig. 1 zeigt eine bevorzugte Ausführungsform der erfindungsgemäßen Messeinrichtung.

Die erfindungsgemäße Messeinrichtung weist ein Klemmelement in Form eines Magneten 6 auf. Das Klemmelement 6 wiederum weist eine Vertiefung auf, in der eine Sensorfassung 5 angeordnet ist, die den oberen, das heißt der Pflanzenprobe zugeneigten Abschluss des Klemmelementes bildet. In der Vertiefung der Sensorfassung ist ein Sensoreinsatz aufgenommen. Der Sensoreinsatz weist eine Leiterplatte bzw. ein Stanzgitter 8 auf, die/das einen Drucksensor 4 trägt. Der Drucksensor 4 ist über Ansteuerleitungen 9 oder über eine drahtlose Verbindung mit einer Auswertungseinrichtung verbunden.

Wie in der vergrößerten Darstellung gezeigt ist, ist der Drucksensor des erfindungsgemäßen Klemmelements in der Vertiefung der Sensorfassung eingegossen. Erfindungsgemäß erfolgt dies vorzugsweise in drei Schritten oder Stufen, die durch die drei Schichten 14, 13 und 12 dargestellt sind. Polymerschicht 14 ist die erste eingebrachte Schicht, die sich an der Wand des Sensoreinsatzes hochzieht. Darauf wird die zweite Polymerschicht 13 aufgebracht, bevor dann die Abschlussschicht 12 vergossen wird.

Zur Entlüftung beim Vergießen ist ein Durchgangskanal 10 vorgesehen. Dieser erstreckt sich durch die Leiterplatte und den Magneten 6, um beim Verfüllen des Vergussmaterials ein Entweichen der in der Ausnehmung vorhandenen Luft zu ermöglichen.

Am unteren Ende, d.h. an der der Pflanzenprobe abgewandten Seite ist vorzugsweise ein Abschlussgehäuse 11 vorgesehen.

Ferner zeigt Fig. 1 das Gegen-Klemmelement mit einem Gegenstempel 3, einem Magneten 2 und einem Stab mit Drehgewinde 1.

Das Klemmelement und das Gegen-Klemmelement sind zueinander gegenüberliegend ausgerichtet und so auf entgegen gesetzten Oberflächen einer Pflanzenprobe positionierbar. Dabei sind die beiden Elemente senkrecht zur Oberfläche der Pflanzenprobe angeordnet.

Über das Drehgewinde 1 kann die Position des Magneten 2 des Gegen-Klemmelements relativ zum Gegenstempel 3 eingestellt werden. Je nach axialer Lage des Magneten 2 am Stab 1 ist die vom Gegen-Klemmelement auf das Klemmelement aufgewandte Kraft stärker oder schwächer. Der Magnet weist dazu beispielsweise eine zentrale Öffnung mit einem Innengewinde auf. So kann der Abstand des Magneten 2 vom Magneten 6 eingestellt werden. Da die magnetische Wechselwirkung zwischen den Magneten von deren gegenseitigen Abstand abhängt, ergibt sich somit durch das Drehen des Magneten 2 eine gezielte Einstellung des Klemmdruckes der Klemmeinrichtung.

Die Kontaktfläche der Sensorfassung des Klemmelementes hat in der gezeigten Ausführungsform die gleiche Form und Größe wie der Gegenstempel 3 des Gegen-Klemmelements. Wie auch immer die Größenverhältnisse sind, es ist darauf zu achten, dass eine Abdeckung der aktiven Sensorfläche gewährleistet ist.

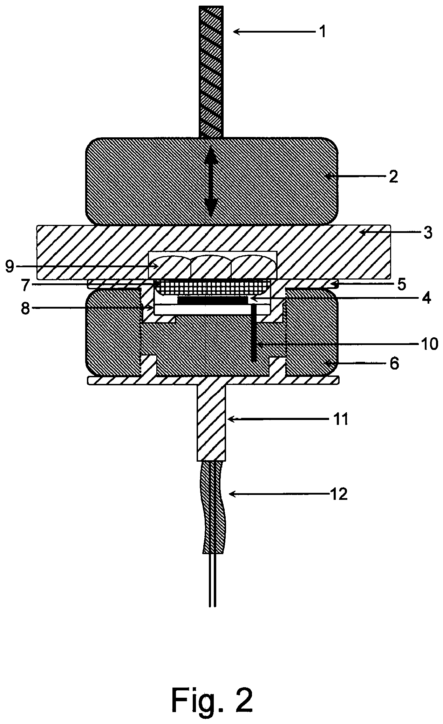

Wie in Fig. 1 gezeigt ist, ist die Oberfläche des Klemmelemente als auch die Oberfläche des Gegenstempels 3 eben ausgebildet. Wie in Fig. 2 beispielhaft gezeigt, sind auch andere Oberflächen denkbar, wie etwa zur Messung an anderen Pflanzenteilen, wie etwa an einem Stängel oder einer Nadel.

Bei der Herstellung des Klemmelementes wird vorzugsweise zunächst die Leiterplatte/Stanzgitter mit dem darauf angeordneten Drucksensor am Boden der Ausnehmung des Magneten 6 eingeklebt. Danach erfolgt ein Einfüllen einer ersten Menge an Vergussmaterial 14 in die Ausnehmung, wobei die erste Menge so bemessen ist, dass lediglich die Leiterplatte/Stanzgitter und der Drucksensor bedeckt sind, sowie ein Hochziehen der Vergussmasse an der unteren Hälfte der Innenwand des Sensorgehäuses stattfinden kann. Nachdem die erste Vergussmaterialmenge mit der Leiterplatte/Stanzgitter, dem Drucksensor und der Innenwand vernetzt und ausgehärtet ist, wird eine zweite Menge an Vergussmaterial 13 derart eingehüllt, dass unter Berücksichtigung der Ausdehnung bei der Vernetzung der zweiten Vergussmaterialmenge mit der Wand der Ausnehmung die zweite Vergussmaterialmenge unterhalb des oberen Randes der Ausnehmung bleibt. Abschließend erfolgt nach Vernetzung der zweiten Vergussmaterialmenge mit der Ausnehmungswand ein Einfüllen einer dritten Menge an Vergussmaterial 12, derart dass ein passgenauer Abschluss des Vergussmaterials 12 mit der ersten Kontaktfläche der Sensorfassung 5 erreicht wird

Fig. 2 zeigt eine andere Ausführungsform der erfindungsgemäßen Messeinrichtung. Der grundsätzliche Aufbau stimmt mit dem von Fig. 1 überein. Allerdings ist in Fig. 2 zu erkennen, dass das Sensor-Klemmelement und insbesondere der Gegenstempel 3 eine Struktur in Form einer Vertiefung aufweist. In der Stufe sind, wie in Fig. 2 gezeigt, Nadeln eines Nadelbaumes aufgenommen. Dies stellt einen guten Kontakt zu den Nadeln her.

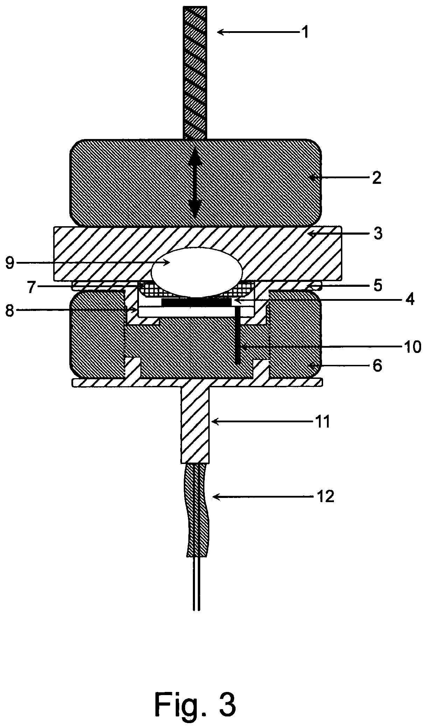

Fig. 3 zeigt eine andere Ausführungsform der erfindungsgemäßen Messeinrichtung. Der grundsätzliche Aufbau stimmt mit dem von Fig. 1 überein. Allerdings ist in Fig. 3 zu erkennen, dass das Gegen-Klemmelement 3 und das Sensor-Klemmelement eine Vertiefung aufweisen, um darin beispielsweise einen Fruchtstiel aufzunehmen.

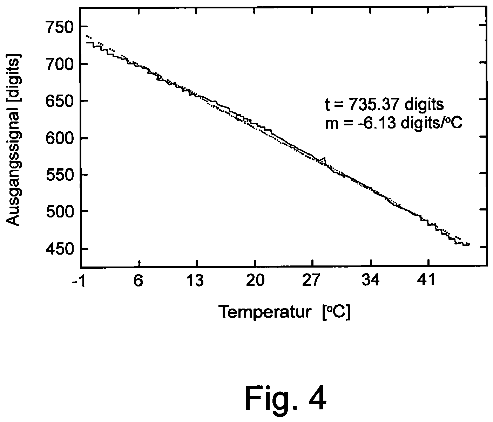

In Fig. 4 ist ein Beispiel einer Eichgeraden für die Temperaturkompensation einer Sonde gezeigt. Aufgetragen sind die Ausgangssignale der Sonden in Digits gegen die Temperatur in Grad Celsius. Aus dieser Regressionsgeraden werden die Steigung (m; im Beispiel m= -6,13 digits/°C) sowie der y-Achsenabschnitt (t; im Beispiel t= 735,37 digits) bestimmt. Um die Temperaturabhängigkeit zu kompensieren, wird wie erläutert V[p] (kompensiertes Ausgangssignal) nach der Gleichung V[p] = V[p, T] - ((T · m) + t) berechnet. Da das Sondensignal gemäß der vorgenannten Gleichung auch vom Druck abhängt, wird anschließend die Druckabhängigkeit der Sonde bestimmt, indem die Sonde gegen den Luftdruck geeicht wird. Das temperaturkompensierte Signal V [p] wird mit einer entsprechenden Software in der Datenbank abgespeichert und entsprechend visualisiert.

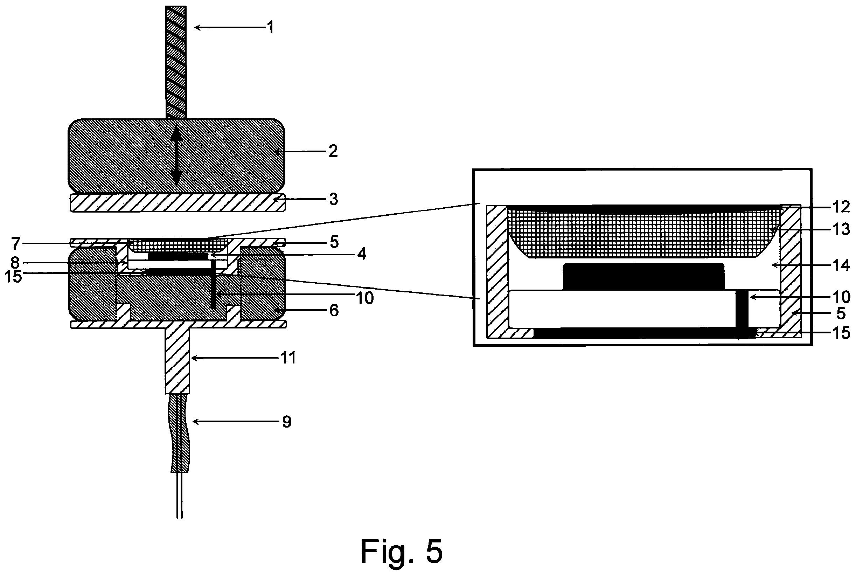

Fig. 5 zeigt eine weitere bevorzugte Ausführungsform der erfindungsgemäßen Messeinrichtung. Die hier gezeigte Messeinrichtung weist wieder ein Klemmelement in Form eines Magneten 6 auf. Das Klemmelement 6 wiederum weist eine Vertiefung auf, in der eine Sensorfassung 5 angeordnet ist, die den oberen, das heißt der Pflanzenprobe zugeneigten Abschluss des Klemmelementes bildet. In der Vertiefung der Sensorfassung ist ein Sensoreinsatz aufgenommen. Der Sensoreinsatz weist eine Leiterplatte bzw. ein Stanzgitter 8 auf, die/das einen Drucksensor 4 trägt. Der Drucksensor 4 ist über Ansteuerleitungen 9 oder über eine drahtlose Verbindung mit einer Auswertungseinrichtung verbunden.

Wie in der vergrößerten Darstellung von Fig. 5 gezeigt ist, ist auch der Drucksensor dieses erfindungsgemäßen Klemmelements in der Vertiefung der Sensorfassung eingegossen. Erfindungsgemäß erfolgt dies vorzugsweise in drei Schritten oder Stufen, die durch die drei Schichten 14, 13 und 12 dargestellt sind. Polymerschicht 14 ist die erste eingebrachte Schicht, die sich an der Wand des Sensoreinsatzes hochzieht. Darauf wird die zweite Polymerschicht 13 aufgebracht, bevor dann die Abschlussschicht 12 vergossen wird.

In dieser Ausführungsform ist unterhalb der Leiterplatteplatte/dem Stanzgitter 8, die den Drucksensor 4 enthält, eine weitere polymere Schicht als Sperrschicht 15 eingebracht, die den Drucksensor 4 samt Leiterplatte 8 gegen die äußere Atmosphäre (Feuchtigkeit, Temperatur etc.) abschirmt. Die Sperrschicht besteht vorzugsweise aus Wärme leitendem Material, wie etwa ein Polymer, eine Membran, Wachs, eine visköse Lösung oder eine wasserabweisende Substanz.

Zur Entlüftung beim Vergießen ist auch bei dieser Ausführungsform ein Durchgangskanal 10 vorgesehen. Dieser erstreckt sich durch die Leiterplatte 8, die Sperrschicht 15 und den Magneten 6, um beim Verfüllen des Vergussmaterials ein Entweichen der in der Ausnehmung vorhandenen Luft zu ermöglichen. Alternativ erstreckt sich der Durchgangskanal nicht durch die Sperrschicht, da es zur Entlüftung als ausreichend angesehen werden kann, wenn beim Einbringen der Sperrschicht vorhandene Luft entweichen kann.

Am unteren Ende, d.h. an der der Pflanzenprobe abgewandten Seite ist vorzugsweise ein Abschlussgehäuse 11 vorgesehen.

Ferner zeigt Fig. 5 das Gegen-Klemmelement mit einem Gegenstempel 3, einem Magneten 2 und einem Stab mit Drehgewinde 1.

Wie in Fig. 5 gezeigt ist, ist die Oberfläche des Klemmelemente als auch die Oberfläche des Gegenstempels 3 eben ausgebildet, wie bereits oben im Zusammenhang mit Fig. 1 erläutert. Wie in Fig. 2 beispielhaft gezeigt, sind jedoch auch für diese Ausführungsform andere Oberflächen denkbar, wie etwa zur Messung an anderen Pflanzenteilen, wie etwa an einem Stängel oder einer Nadel.

Bei der Herstellung des Klemmelementes wird zunächst die Sperrschicht am Boden der Ausnehmung des Magneten 6 ausgebildet. Anschließend werden zunächst die Leiterplatte/Stanzgitter mit dem darauf angeordneten Drucksensor am Boden der Ausnehmung des Magneten 6 eingeklebt. Danach erfolgt ein Einfüllen einer ersten Menge an Vergussmaterial 14 in die Ausnehmung, wobei die erste Menge so bemessen ist, dass lediglich die Leiterplatte/Stanzgitter und der Drucksensor bedeckt sind, sowie ein Hochziehen der Vergussmasse an der unteren Hälfte der Innenwand des Sensorgehäuses stattfinden kann. Nachdem die erste Vergussmaterialmenge mit der Leiterplatte/Stanzgitter, dem Drucksensor und der Innenwand vernetzt und ausgehärtet ist, wird eine zweite Menge an Vergussmaterial 13 derart eingefüllt, dass unter Berücksichtigung der Ausdehnung bei der Vernetzung der zweiten Vergussmaterialmenge mit der Wand der Ausnehmung die zweite Vergussmaterialmenge unterhalb des oberen Randes der Ausnehmung bleibt. Abschließend erfolgt nach Vernetzung der zweiten Vergussmaterialmenge mit der Ausnehmungswand ein Einfüllen einer dritten Menge an Vergussmaterial 12, derart dass ein passgenauer Abschluss des Vergussmaterials 12 mit der ersten Kontaktfläche der Sensorfassung 5 erreicht wird.

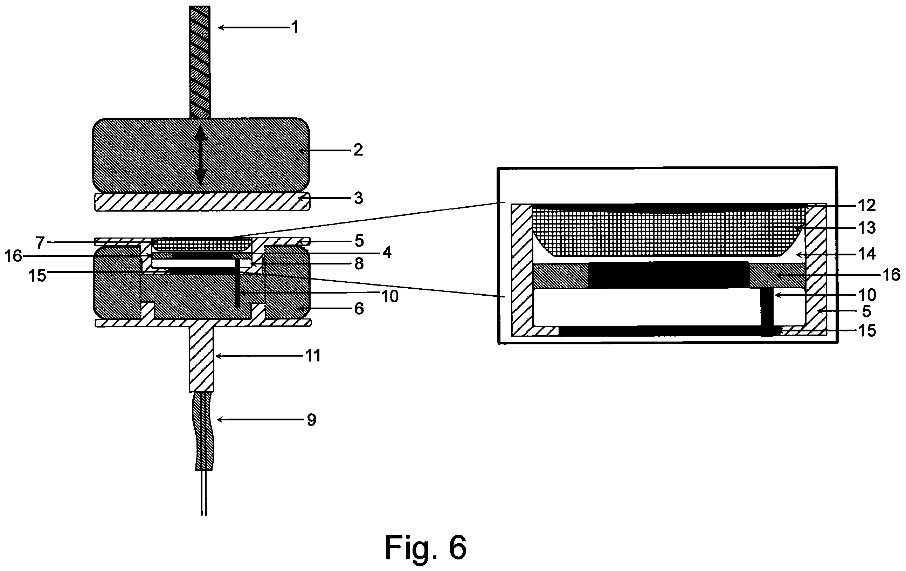

Die in Fig. 6 gezeigte Ausführungsform; entspricht im Wesentlichen den oben beschriebenen Ausführungsformen. Um Wiederholungen zu vermeiden, werden im Folgenden nur die Unterscheidungsmerkmale beschrieben.

Bei der Ausführungsform von Fig. 6 ist der Drucksensor zusätzlich mit einem aushärtenden Polymer umhüllt. Dies ist in Fig. 6 mit Bezugszeichen 16 bezeichnet. Dieses Polymer (z.B. Hysol FP4451TD) wird in die Ausnehmung im Magneten eingeführt, bevor der Raum darüber mit Silikon bzw. einem anderen Polymer in den beschriebenen drei oder mehr Schritten ausgefüllt wird. Das Polymer wird dabei bis zur Oberkante des Sensors eingebracht. Dies stellt sicher, dass Ecken und Kanten immer gleichmäßig von dem dann einzutragenden Silikon oder Polymer ausgefüllt werden und die Polymerisation in solch kritischen Bereichen gleichmäßig bzw. vollständig abläuft.

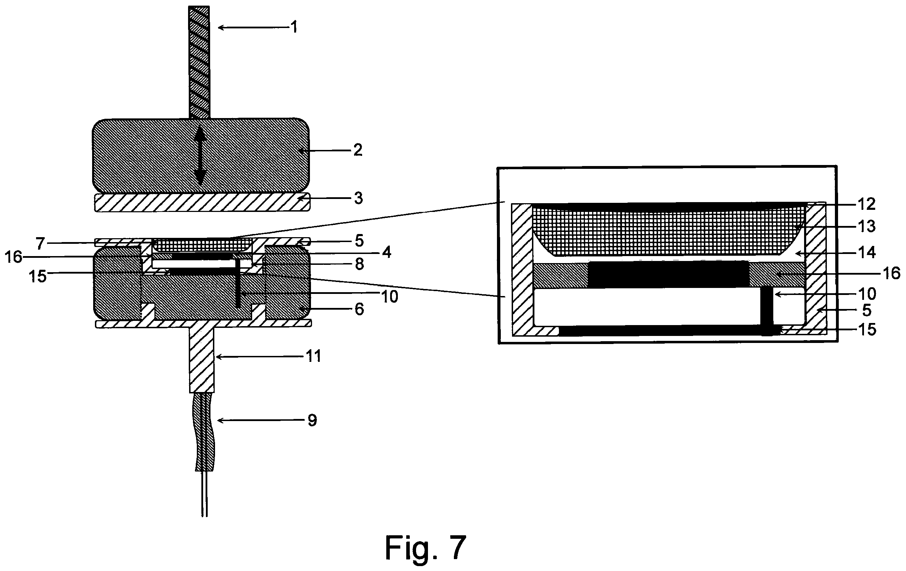

Eine weitere Modifikation der Messeinrichtung ist in Fig. 7 gezeigt. Hier ist die Innenwandung der Sensorfassung 5 konusförmig ausgebildet, mit einem zur Öffnung hin kleiner werdenden Durchmesser. Eine solche Verjüngung ist für alle erläuterten erfindungsgemäßen Ausführungsformen verwendbar, um einen besseren Halt der obersten Polymerschicht zu gewährleisten. Ein Abreißen der letzten Schicht wird dadurch noch besser verhindert.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend nicht genannt sind.

Im Weiteren schließt der Ausdruck "umfassen" und Ableitungen davon andere Elemente oder Schritte nicht aus. Ebenfalls schließt der unbestimmte Artikel "ein" bzw. "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

## Patentansprüche

1. Verfahren zur Herstellung eines Klemmelements für eine Turgördruck-Messeinrichtung zum Messen des Turgordrucks in einer Pflanzenprobe, wobei das Klemmelement aufweist:
- ein Sensoraufnahmeteil (5), mit einer ersten Kontaktfläche, einer der Kontaktfläche gegenüberliegenden Fläche und einer diese verbindenden Umfangsfläche, wobei das Klemmelement eine Längsachse aufweist, die senkrecht zur ersten Kontaktfläche verläuft, und mit einer Ausnehmung auf der Seite der ersten Kontaktfläche, die zur ersten Kontaktfläche hin offen ist;
- einen auf einer Leiterplatte/Stanzgitter (8) angeordneten Drucksensor (4) zur Messung eines Druckantwortsignals der Pflanzenprobe, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt;
**gekennzeichnet durch** die Schritte:
(a) Befestigen der Leiterplatte/Stanzgitter mit dem darauf angeordneten Drucksensor am Boden oder an der Wandung der Ausnehmung;
(b) Einfüllen einer ersten Menge (14) an Vergussmaterial in die Ausnehmung, wobei die erste Menge so bemessen ist, dass lediglich die Leiterplatte/Stanzgitter und der Drucksensor bedeckt sind, sowie ein Hochziehen der Vergussmasse an der unteren Hälfte der Innenwand des Sensorgehäuses stattfinden kann;
(c) nach Vernetzung der ersten Vergussmaterialmenge mit der Leiterplatte/Stanzgitter, dem Drucksensor und der Innenwand, Einfüllen einer zweiten Menge (13) an Vergussmaterial derart, dass unter Berücksichtigung der Ausdehnung bei der Vernetzung der zweiten Vergussmaterialmenge mit der Wand der Ausnehmung die zweite Vergussmaterialmenge unterhalb des oberen Randes der Ausnehmung bleibt; und
(d) nach Vernetzung der zweiten Vergussmaterialmenge mit der Ausnehmungswand, Einfüllen einer dritten Menge (12) an Vergussmaterial, derart dass ein passgenauer Abschluss des Vergussmaterials mit der ersten Kontaktfläche erreicht wird.

2. Verfahren nach Anspruch 1, ferner mit dem Schritt, vor dem vollständigen Aushärten der dritten Vergussmaterialmenge, Ausbilden einer negativen Abschlussstruktur auf der Oberfläche des Vergussmaterials entsprechend einer pflanzlichen Oberflächenstruktur.

3. Verfahren nach Anspruch 2, wobei die negative Abschlussstruktur auf der Oberfläche des Vergussmaterials eingeprägt wird.

4. Verfahren nach Anspruch 1, ferner mit dem Schritt, nach dem vollständigen Aushärten der dritten Vergussmaterialmenge, Ausbilden einer negativen Abschlussstruktur auf der Oberfläche des Vergussmaterials entsprechend einer pflanzlichen Oberflächenstruktur.

5. Verfahren nach Anspruch 3 oder 4, wobei die Abschlussstruktur mittels eines Gegenklemmelements der Turgordruck-Messeinrichtung, die eine entsprechende Gegenstruktur aufweist, erzeugt wird.

6. Klemmelement für eine Turgordruck-Messeinrichtung zum Messen des Turgordrucks in einer Pflanzenprobe, hergestellt mit dem Verfahren nach einem der Ansprüche 1 bis 5.

7. Turgordruck-Messeinrichtung zum Messen des Turgordrucks in einer Pflanzenprobe, mit:
(a) einem ersten Klemmelement, das aufweist:
- ein Sensoraufnahmeteil (5), mit einer ersten Kontaktfläche, einer der Kontaktfläche gegenüberliegenden Fläche und einer diese verbindenden Umfangsfläche, wobei das Klemmelement eine Längsachse aufweist, die senkrecht zur ersten Kontaktfläche verläuft, und mit einer Ausnehmung auf der Seite der ersten Kontaktfläche, wobei die Ausnehmung zur ersten Kontaktfläche hin offen ist;
- ein erstes Kraftelement (6), das auf der der ersten Kontaktfläche gegenüberliegenden Fläche des Sensoraufnahmeteils angeordnet ist;
- einen auf einer Leiterplatte/Stänzgitter (8) angeordneten Drucksensor (4) zur Messung eines Druckantwortsignals der Pflanzenprobe, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt;
- wobei die Ausnehmung mit einem ausgehärteten Vergussmaterial (12, 13, 14) mit homogenem Abschluss an der ersten Kontaktfläche verfüllt ist;
(b) einem zweiten Klemmelement, das aufweist:
- ein Gegenklemmteil mit einer zweiten Kontaktfläche, einer der zweiten Kontaktfläche gegenüberliegenden Fläche und einer diese miteinander verbindenden Umfangsfläche;
- ein zweites Kraftelement (2), das auf der der zweiten Kontaktfläche gegenüberliegenden Seite angeordnet ist;
wobei eines der beiden Kraftelemente einen Magneten aufweist; und
wobei die Turgordruck-Messeinrichtung als Ganzes temperaturkompensiert ist.

8. Turgordruck-Messeinrichtung nach Anspruch 7, wobei das Klemmelement als Ganzes mittels linearer Regression temperaturkompensiert ist.

9. Turgordruck-Messeinrichtung nach Anspruch 7 oder 8, wobei das Sensoraufnahmeteil einen Durchgangskanal aufweist, der sich vom Inneren der Ausnehmung zu einer Außenfläche erstreckt, und wobei die Leiterplatte/Stanzgitter einen Durchgangskanal (10) aufweist, der von der der Ausnehmungsöffnung zugewandten Seite der Leiterplatte/Stanzgitter (8) zu der dem Boden der Ausnehmung zugewandten Seite verläuft, wobei die Öffnung des Durchgangskanals auf der dem Boden zugewandten Seite mit der inneren Öffnung des Durchgangskanals des Sensoraufnahmeteils fluchtet.

10. Turgordruck-Messeinrichtung nach Anspruch 9, wobei die Leiterplatte/Stanzgitter (8) mindestens zwei Durchgangskanäle aufweist, die von der der Ausnehmungsöffnung zugewandten Seite der Leiterplatte/Stanzgitter zu der dem Boden der Ausnehmung zugewandten Seite der Leiterplatte/Stanzgitter verlaufen.

11. Turgordruck-Messeinrichtung nach einem der Ansprüche 7 bis 9, wobei das Vergussmaterial ein vernetzbares Polymer aufweist oder aus vernetzbarem Polymer besteht.

12. Turgordruck-Messeinrichtung nach Anspruch 11, wobei das Vergussmaterial ausgewählt ist aus Epoxydharz, Silikon, additiv vernetzendem Silikon, kondensierend vernetzendem Silikon, organischem Hydrogel oder natürlichem Hydrogel mit einer Wärmeleitfähigkeit >0,4 Watt x m⁻¹ x K⁻¹.

13. Turgordruck-Messeinrichtung nach einem der Ansprüche 7 bis 12, wobei in dem Sensoraufnahmeteil (5) zwischen dem Boden oder der Wandung der Ausnehmung und der dem Boden / Wandung zugewandten Seite der Leiterplatte/Stanzgitter (8) eine Klebstoffschicht ist.

14. Turgordruck-Messeinrichtung nach Anspruch 11 oder 13, wobei das Material des Klebstoffes einen ähnlichen Ausdehnungskoeffizienten aufweist, wie das Vergussmaterial.

15. Turgordruck-Messeinrichtung nach einem der Ansprüche 7 bis 14, wobei das Vergussmaterial undurchlässig ist für ultraviolette Strahlung.

16. Turgordruck-Messeinrichtung nach einem der Ansprüche 7 bis 15, wobei die zweite Kontaktfläche des zweiten Klemmelements strukturiert ist.

17. Verfahren zum Betreiben einer Turgordruck-Messeinrichtung zum Messen des Turgordrucks in einer Pflanzenprobe, mit
(a) einem ersten Klemmelement, das aufweist:
- ein Sensoraufnahmeteil (5), mit einer ersten Kontaktfläche, einer der Kontaktfläche gegenüberliegenden Fläche und einer diese verbindenden Umfangsfläche, wobei das Klemmelement eine Längsachse aufweist, die senkrecht zur ersten Kontaktfläche verläuft, und mit einer Ausnehmung auf der Seite der ersten Kontaktfläche, wobei die Ausnehmung zur ersten Kontaktfläche hin offen ist;
- ein erstes Kraftelement (6), das auf der der ersten Kontaktfläche gegenüberliegenden Fläche des Sensoraufnahmeteils angeordnet ist;
- einen auf einer Leiterplatte/Stanzgitter (8) angeordneten Drucksensor (4) zur Messung eines Druckantwortsignals der Pflanzenprobe, wobei die Leiterplatte/Stanzgitter mit dem Drucksensor derart in der Ausnehmung des Sensoraufnahmeteils angeordnet ist, dass der Drucksensor der ersten Kontaktfläche zugewandt ist und die Leiterplatte/Stanzgitter auf dem Boden der Ausnehmung liegt;
- wobei die Ausnehmung mit einem ausgehärteten Vergussmaterial (12, 13, 14) mit homogenem Abschluss an der ersten Kontaktfläche verfüllt ist;
(b) einem zweiten Klemmelement, das aufweist:
- ein Gegenklemmteil mit einer zweiten Kontaktfläche, einer der zweiten Kontaktfläche gegenüberliegenden Fläche und einer diese miteinander verbindenden Umfangsfläche;
- ein zweites Kraftelement (2), das auf der der zweiten Kontaktfläche gegenüberliegenden Seite angeordnet ist;
wobei das Verfahren die Schritte aufweist:
abwechselndes Messen eines Druckantwortsignals von der Pflanzenprobe in einem Druckmessbetriebsmodus der Messeinrichtung und Messen der Temperatur der Oberfläche der Pflanzenprobe in einem Temperaturbetriebsmodus der Messeinrichtung.

18. Verfahren nach Anspruch 17, wobei der Druckmesssensor abwechselnd in einem Druckmessbetriebsmodus und einem Temperaturmessbetriebsmodus betrieben wird.

19. Verfahren nach Anspruch 18, wobei kontinuierlich zwischen dem Druckmessbetriebsmodus und dem Temperaturmessbetriebsmodus umgeschaltet wird.

## Claims

1. A method for producing a clamp element for a turgor pressure measurement device for measuring the turgor pressure in a plant sample, wherein the clamp element comprises:
- a sensor receiving portion (5) with a first contact surface, a surface lying opposite the contact surface, and a peripheral surface connecting the two, wherein the clamp element has a longitudinal axis extending perpendicularly to the first contact surface, and with a recess on the side of the first contact surface, said recess being open towards the first contact surface;
- a pressure sensor (4), which is arranged on a printed circuit board/lead frame (8), to measure a pressure response signal of the plant sample, wherein the printed circuit board/lead frame is arranged with the pressure sensor in the recess of the sensor receiving portion such that the pressure sensor faces the first contact surface and the printed circuit board/lead frame lies at the bottom of the recess,
**characterized by** the steps:
(a) fixing the printed circuit board/lead frame with the pressure sensor arranged thereon at the bottom or at the wall of the recess;
(b) filling a first amount (14) of potting material into the recess, wherein the first amount is selected such that only the printed circuit board/lead frame and the pressure sensor are covered and the potting material can be raised at the lower half of the inner wall of the sensor housing;
(c) after cross-linking of the first amount of potting material with the printed circuit board/lead frame, the pressure sensor and the inner wall, filling in a second amount (13) of potting material such that, taking into account the expansion during cross-linking of the second amount of potting material with the wall of the recess, the second amount of potting material remains below the upper edge of the recess; and
(d) after cross-linking of the second amount of potting material with the recess wall, filling in a third amount (12) of potting material such that the top of the potting material lies flush with the first contact surface.

2. The method according to claim 1, further comprising the step: before completely curing of the third amount of potting material, forming a negative top structure on the surface of the potting material in accordance with a surface structure of a plant.

3. The method according to claim 2, wherein the negative top structure is stamped into the surface of the potting material.

4. The method according to claim 1, further comprising the step: after completely curing of the third amount of potting material, forming a negative top structure on the surface of the potting material in accordance with a surface structure of a plant.

5. The method according to claim 3 or 4, wherein the top structure is formed by means of a counter clamp element of the turgor pressure measurement device having a corresponding counter structure.

6. A clamp element for a turgor pressure measurement device for measuring the turgor pressure in a plant sample, produced in accordance with the method according to any one of claims 1 to 5.

7. A turgor pressure measurement device for measuring the turgor pressure in a plant sample, comprising:
(a) a first clamp element comprising:
- a sensor receiving portion (5) with a first contact surface, a surface lying opposite the contact surface, and a peripheral surface connecting the two, wherein the clamp element has a longitudinal axis extending perpendicularly to the first contact surface, and with a recess on the side of the first contact surface, wherein the recess is open towards the first contact surface;
- a first force element (6) which is arranged on the surface of the sensor receiving portion opposite the first contact surface;
- a pressure sensor (4), which is arranged on the printed circuit board/lead frame (8), to measure a pressure response signal of the plant sample, the printed circuit board/lead frame with the pressure sensor being arranged in the recess of the sensor receiving portion such that the pressure sensor faces the first contact surface and the printed circuit board/lead frame lies at the bottom of the recess;
- wherein the recess is filled with a cured potting material (12, 13, 14) with homogeneous top at the first contact surface;
(b) a second clamp element comprising:
- a counter clamp part with a second contact surface, a surface lying opposite the second contact surface, and a peripheral surface connecting the two;
- a second force element (2) arranged on the side opposite the second contact surface;
wherein one of the two force elements has a magnet; and
wherein the turgor pressure measurement device is temperature-compensated as a whole.

8. The turgor pressure measurement device according to claim 7, wherein the clamp element is temperature-compensated as a whole by linear regression.

9. The turgor pressure measurement device according to claim 7 or 8, wherein the sensor receiving portion comprises a through channel extending from the interior of the recess to an outer surface, and wherein the printed circuit board/lead frame comprises a through channel (10) extending from the side of the printed circuit board/lead frame (8) facing the recess opening to the side facing the bottom of the recess, wherein the opening of the through channel on the side facing the bottom is flush with the inner opening of the through channel of the sensor receiving portion.

10. The turgor pressure measurement device according to claim 9, wherein the printed circuit board/lead frame (8) comprises at least two through channels extending from the side of the printed circuit board/lead frame facing the recess opening to the side of the printed circuit board/lead frame facing the bottom of the recess.

11. The turgor pressure measurement device according to any one of claims 7 to 9, wherein the potting material comprises a cross-linkable polymer or consists of a cross-linkable polymer.

12. The turgor pressure measurement device according to claim 11, wherein the potting material is selected from epoxy resin, silicone, additively cross-linking silicone, silicone cross-linking in a condensing manner, organic hydrogel or natural hydrogel having a thermal conductivity of >0.4 Watt x m⁻¹ x K⁻¹.

13. The turgor pressure measurement device according to any one of claims 7 to 12, wherein the sensor receiving portion (5) comprises an adhesive layer between the bottom or the wall of the recess and the side of the printed circuit board/lead frame (8) facing the bottom/wall.

14. The turgor pressure measurement device according to claim 11 or 13, wherein the material of the adhesive has an expansion coefficient which is similar to that of the potting material.

15. The turgor pressure measurement device according to any one of claims 7 to 14, wherein the potting material is not transparent to ultraviolet radiation.

16. The turgor pressure measurement device according to any one of claims 7 to 15, wherein the second contact surface of the second clamp element is structured.

17. A method for operating a turgor pressure measurement device for measuring the turgor pressure of a plant sample, comprising:
(a) a first clamp element which comprises:
- a sensor receiving portion (5) with a first contact surface, a surface lying opposite the contact surface, and a peripheral surface connecting the two, wherein the clamp element has a longitudinal axis extending perpendicularly to the first contact surface, and with a recess on the side of the first contact surface, wherein the recess is open towards the first contact surface;
- a first force element (6) which is arranged on the surface of the sensor receiving portion which is opposite the first contact surface;
- a pressure sensor (4), which is arranged on a printed circuit board/lead frame (8), to measure a pressure response signal of the plant sample, the printed circuit board/lead frame being arranged with the pressure sensor in the recess of the sensor receiving portion such that the pressure sensor faces the first contact surface and the printed circuit board/lead frame lies at the bottom of the recess;
- wherein the recess is filled with a cured potting material (12, 13, 14) with a homogeneous top at the first contact surface;
(b) a second clamp element which comprises:
- a counter clamp part with a second contact surface, a surface lying opposite the second contact surface, and a peripheral surface connecting the two;
- a second force element (2) which is arranged on the side opposite the second contact surface;
wherein the method comprises the steps:
alternately measuring a pressure response signal of the plant sample in a pressure measurement mode of the measurement device and measuring the temperature of the surface of the plant sample in a temperature mode of the measurement device.

18. The method according to claim 17, wherein the pressure measurement sensor is operated alternately in a pressure measurement mode and a temperature measurement mode.

19. The method according to claim 18, wherein it is shifted continuously between the pressure measurement mode and the temperature measurement mode.

## Revendications

1. Procédé de fabrication d'un élément de serrage pour un dispositif de mesure de pression de turgescence destiné à mesurer la turgescence dans un échantillon végétal, ledit élément de serrage comprenant :
- un élément de logement de capteur (5) avec une première surface de contact, une surface opposée à la surface de contact et une surface périphérique reliant celles-ci, l'élément de serrage présentant un axe longitudinal s'étendant perpendiculairement à la première surface de contact, et avec un évidement sur le côté de la première surface de contact, lequel s'ouvre sur la première surface de contact ;
- un capteur de pression (4) disposé sur une carte de circuit imprimé/grille estampée (8) pour mesurer un signal de réponse de pression de l'échantillon végétal, ladite carte de circuit imprimé/grille estampée étant disposée avec le capteur de pression dans l'évidement de l'élément de logement de capteur de telle manière que le capteur de pression fait face à la première surface de contact et la carte de circuit imprimé/grille estampée repose sur le fond de l'évidement ;
**caractérisé par** les étapes suivantes :
(a) fixation de la carte de circuit imprimé/grille estampée avec le capteur de pression disposé sur celle-ci sur le fond ou sur la paroi de l'évidement ;
(b) versement d'une première quantité (14) de matériau de scellement dans l'évidement, ladite première quantité étant mesurée de manière à ne recouvrir que la carte de circuit imprimé/grille estampée et le capteur de pression, et à permettre la remontée de la masse de scellement sur la moitié inférieure de la paroi intérieure du boîtier de capteur ;
(c) après réticulation de la première quantité de matériau de scellement avec la carte de circuit imprimé/grille estampée, le capteur de pression et la paroi intérieure, versement d'une deuxième quantité (13) de matériau de scellement de sorte que, compte tenu de la dilatation à la réticulation de la deuxième quantité de matériau de scellement avec la paroi de l'évidement, la deuxième quantité de matériau de scellement reste en dessous du bord supérieur de l'évidement ; et
(d) après réticulation de la deuxième quantité de matériau de scellement avec la paroi d'évidement, versement d'une troisième quantité (12) de matériau de scellement, de manière à obtenir une bordure ajustée du matériau de scellement avec la première surface de contact.

2. Procédé selon la revendication 1, comprenant en outre l'étape, antérieure au durcissement complet de la troisième quantité de matériau de scellement, de formation d'une structure de bordure négative sur la surface du matériau de scellement en fonction d'une structure de surface végétale.

3. Procédé selon la revendication 2, où la structure de bordure négative est imprimée sur la surface du matériau de scellement.

4. Procédé selon la revendication 1, comprenant en outre l'étape, postérieure au durcissement complet de la troisième quantité de matériau de scellement, de formation d'une structure de bordure négative sur la surface du matériau de scellement en fonction d'une structure de surface végétale.

5. Procédé selon la revendication 3 ou la revendication 4, où la structure de bordure est générée au moyen d'un contre-élément de serrage du dispositif de mesure de pression de turgescence, lequel présente une contre-structure correspondante.

6. Elément de serrage pour un dispositif de mesure de pression de turgescence destiné à mesurer la turgescence dans un échantillon végétal, fabriqué suivant le procédé selon l'une des revendications 1 à 5.

7. Dispositif de mesure de pression de turgescence destiné à mesurer la turgescence dans un échantillon végétal, avec :
(a) un premier élément de serrage, comprenant :
- un élément de logement de capteur (5), avec une première surface de contact, une surface opposée à la surface de contact et une surface périphérique reliant celles-ci, l'élément de serrage présentant un axe longitudinal s'étendant perpendiculairement à la première surface de contact, et avec un évidement sur le côté de la première surface de contact, lequel s'ouvre sur la première surface de contact ;
- un premier élément d'application de force (6) disposé sur la surface de l'élément de logement de capteur opposée à la première surface de contact ;
- un capteur de pression (4) disposé sur une carte de circuit imprimé/grille estampée (8) pour mesurer un signal de réponse de pression de l'échantillon végétal, ladite carte de circuit imprimé/grille estampée étant disposée avec le capteur de pression dans l'évidement de l'élément de logement de capteur de telle manière que le capteur de pression fait face à la première surface de contact et la carte de circuit imprimé/grille estampée repose sur le fond de l'évidement ;
- l'évidement étant rempli avec un matériau de scellement (12, 13, 14) durci ayant une bordure homogène sur la première surface de contact ;
(b) un deuxième élément de serrage, comprenant :
- une partie de contre-serrage avec une deuxième surface de contact, une surface opposée à la deuxième surface de contact et une surface périphérique reliant celles-ci ;
- un deuxième élément d'application de force (2) disposé sur le côté opposé à la deuxième surface de contact ;
une des deux éléments d'application de force étant pourvu d'un aimant ; et
le dispositif de mesure de pression de turgescence étant soumis à une compensation de température dans son ensemble.

8. Dispositif de mesure de pression de turgescence selon la revendication 7, où l'élément de serrage est soumis à une compensation de température par régression linéaire dans son ensemble.

9. Dispositif de mesure de pression de turgescence selon la revendication 7 ou la revendication 8, où l'élément de logement de capteur présente un canal de traversée qui s'étend de l'intérieur de l'évidement vers une surface extérieure, et où la carte de circuit imprimé/grille estampée présente un canal de traversée (10) qui s'étend du côté de la carte de circuit imprimé/grille estampée (8) faisant face à l'ouverture d'évidement vers le côté faisant face au fond de l'évidement, l'ouverture du canal de traversée sur le côté faisant face au fond étant alignée avec l'ouverture intérieure du canal de traversée de l'élément de logement de capteur.

10. Dispositif de mesure de pression de turgescence selon la revendication 9, où la carte de circuit imprimé/grille estampée (8) présente au moins deux canaux de traversée s'étendant du côté de la carte de circuit imprimé/grille estampée faisant face à l'ouverture d'évidement vers le côté de la carte de circuit imprimé/grille estampée faisant face au fond de l'évidement.

11. Dispositif de mesure de pression de turgescence selon l'une des revendications 7 à 9, où le matériau de scellement comprend un polymère réticulable ou consiste en un polymère réticulable.

12. Dispositif de mesure de pression de turgescence selon la revendication 11, où le matériau de scellement est sélectionné entre résine époxy, silicone, silicone réticulant par addition, silicone réticulant par condensation, hydrogel organique ou hydrogel naturel avec une conductivité thermique >0,4 W x m⁻¹ x K⁻¹.

13. Dispositif de mesure de pression de turgescence selon l'une des revendications 7 à 12, où une couche d'adhésif est présente dans l'élément de logement de capteur (5) entre le fond ou la paroi de l'évidement et le côté de la carte de circuit imprimé/grille estampée (8) faisant face au fond / à la paroi.

14. Dispositif de mesure de pression de turgescence selon la revendication 11 ou la revendication 13, où le matériau adhésif a un coefficient de dilatation identique à celui du matériau de scellement.

15. Dispositif de mesure de pression de turgescence selon l'une des revendications 7 à 14, où le matériau de scellement est imperméable au rayonnement ultraviolet.

16. Dispositif de mesure de pression de turgescence selon l'une des revendications 7 à 15, où la deuxième surface de contact du deuxième élément de serrage est structurée.

17. Procédé de fonctionnement d'un dispositif de mesure de pression de turgescence destiné à mesurer la turgescence dans un échantillon végétal, avec
(a) un premier élément de serrage, comprenant :
- un élément de logement de capteur (5), avec une première surface de contact, une surface opposée à la surface de contact et une surface périphérique reliant celles-ci, l'élément de serrage présentant un axe longitudinal s'étendant perpendiculairement à la première surface de contact, et avec un évidement sur le côté de la première surface de contact, lequel s'ouvre sur la première surface de contact ;
- un premier élément d'application de force (6), disposé sur la surface de l'élément de logement de capteur opposée à la première surface de contact ;
- un capteur de pression (4) disposé sur une carte de circuit imprimé/grille estampée (8) pour mesurer un signal de réponse de pression de l'échantillon végétal, ladite carte de circuit imprimé/grille estampée étant disposée avec le capteur de pression dans l'évidement de l'élément de logement de capteur de telle manière que le capteur de pression fait face à la première surface de contact et la carte de circuit imprimé/grille estampée repose sur le fond de l'évidement ;
- l'évidement étant rempli avec un matériau de scellement (12, 13, 14) durci ayant une bordure homogène sur la première surface de contact ;
(b) un deuxième élément de serrage, comprenant :
- une partie de contre-serrage avec une deuxième surface de contact, une surface opposée à la deuxième surface de contact et une surface périphérique reliant celles-ci ;
- un deuxième élément d'application de force (2), disposé sur le côté opposé à la deuxième surface de contact ;
ledit procédé comprenant les étapes suivantes :
mesures alternées d'un signal de réponse de pression de l'échantillon végétal dans un mode de service de mesure de pression du dispositif de mesure, et de la température de la surface de l'échantillon végétal dans un mode de service de mesure de température du dispositif de mesure.

18. Procédé selon la revendication 17, où le capteur de mesure de pression est mis en service en alternance dans un mode de service de mesure de pression et dans un mode de service de mesure de température.

19. Procédé selon la revendication 18, où la commutation est continue entre le mode de service de mesure de pression et le mode de service de mesure de température.
